(19) Europäisches Patentamt
European Patent Office
Office européen des brevets

(11) EP 0 775 479 B1

(12) **FASCICULE DE BREVET EUROPEEN**

(45) Date de publication et mention de la délivrance du brevet:
**28.10.1998 Bulletin 1998/44**

(51) Int. Cl.[6]: **A61K 7/42**, A61K 7/00, A61K 9/127

(21) Numéro de dépôt: **96402196.8**

(22) Date de dépôt: **15.10.1996**

(54) **Composition comprenant une dispersion aqueuse de vésicules lipidiques encapsulant un filtre UV à fonction acide et utilisations en application topique**

Zusammensetzung, enthaltend eine wässrige Dispersion von einen säurefunktionellen UV-Filter enthaltenden Lipidvesikeln zur topischen Anwendung

Composition comprising an aqueous dispersion of lipid vesicles encapsulating an acid-functional UV-filter and its use in topical application

(84) Etats contractants désignés:
**DE ES FR GB IT**

(30) Priorité: **22.11.1995 FR 9513876**

(43) Date de publication de la demande:
**28.05.1997 Bulletin 1997/22**

(73) Titulaire: **L'OREAL**
**75008 Paris (FR)**

(72) Inventeurs:
- **Simonnet, Jean-Thierry**
  **75011 Paris (FR)**
- **Legret, Sylvie**
  **92320 Chatillon (FR)**
- **Ribier, Alain**
  **Décédé (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**EP-A- 0 390 682**
**EP-A- 0 559 502**
**EP-A- 0 582 503**
**DE-A- 2 407 733**
**FR-A- 2 408 387**
**GB-A- 2 025 957**
**GB-A- 2 121 801**
**GB-A- 2 185 019**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 775 479 B1

## Description

La présente invention a trait à une composition comprenant une dispersion aqueuse de vésicules lipidiques encapsulant au moins un agent filtrant les radiations UV comportant une fonction acide et à ses utilisations en application topique notamment dans les formulations cosmétiques ou dermatologiques pour la protection de la peau, du cuir chevelu, des cheveux, des cils ou des sourcils contre les rayons UV.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

On connait dans l'état de la technique des compositions à base de composés actifs comportant une fonction acide, libre ou au moins partiellement neutralisée, filtrant les rayons UV. On connait en particulier certains composés sulfoniques pour leurs bonnes propriétés filtrantes des radiations lumineuses de longueur d'onde comprise entre 280 et 400 nm et plus particulièrement entre 280 et 360 nm, leur stabilité thermique et leur stabilité photochimique.

Pour améliorer le confort d'utilisation de ces actifs à fonction acide (douceur, émollience, facilité d'application) et pour potentialiser leur effet filtrant par rapport aux formes galéniques classiques les contenant, on a proposé de les incorporer dans certains types de systèmes de vésicules lipidiques notamment dans les systèmes de vésicules lipidiques constitués de lipides amphiphiles ioniques naturels dont la chaîne hydrocarbonée est insaturée tels que la lécithine de soja ou la lécithine de tournesol ou dans les systèmes de vésicules constitués de lipides amphiphiles non-ioniques et de lipides amphiphiles ioniques acides tels que le système lauryléther de polyglycéryle/dimirystylphosphate ou le système hexadécyléther de polyglycéryle/dicétylphosphate.

Les systèmes de vésicules constitués de lipides amphiphiles ioniques naturels dont la chaîne hydrocarbonée est insaturée, présentent un taux d'encapsulation beaucoup trop faible pour incorporer dans une quantité suffisamment efficace les agents filtrants à fonction acides.

Les systèmes de vésicules constitués de lipides amphiphiles non-ioniques et de lipides amphiphiles ioniques acides encapsulant un agent filtrant à fonction acide présentent l'inconvénient de conduire instantanément à des pertes importantes du pouvoir filtrant des radiations UV de l'actif (jusqu'à 50%).

On connaît par ailleurs, par le document EP582503, une composition formée d'une dispersion aqueuse de vésicules lipidiques dont la membrane est constituée d'une part de lipides non ioniques amphiphiles particuliers constitués d'esters de polyol et d'acides gras, et d'autre part de lipides ioniques amphiphiles.

La présente invention se propose donc de mettre en oeuvre un système stable de vésicules lipidiques permettant d'encapsuler au moins un composé actif filtrant les rayons UV et comportant au moins une fonction acide, libre ou au moins partiellement neutralisées, dans une quantité suffisamment efficace et de façon durable et sans diminuer le pouvoir filtrant de l'actif ainsi encapsulé.

La demanderesse a découvert de façon inattendue que l'encapsulation de composés actifs filtrant les rayons UV et comportant au moins une fonction acide, libre ou au moins partiellement neutralisée, par des vésicules lipidiques dont la membrane lipidique est formée à partir d'au moins un lipide amphiphile non-ionique ou d'au moins un lipide amphiphile ionique hydrocarboné saturé dont l'indice d'iode est inférieur à 10 et d'au moins un lipide amphiphile ionique totalement neutralisé, permettait de résoudre les problèmes techniques évoqués ci-dessus et d'atteindre les objectifs proposés ci-dessus.

Les vésicules lipidiques selon l'invention encapsulant des composés actifs filtrant les rayons UV et comportant une fonction acide, permettent également d'améliorer la répartition de ces actifs sur la surface des matières kératiniques à traiter, d'améliorer leur persistance et leur rémanence et de potentialiser leur effet filtrant par rapport aux formes galéniques classiques les contenant.

Au sens de la présente invention, la rémanence s'entend de la stabilité au cours du temps de l'indice de protection dans l'UVA et/ou l'UVB d'une composition antisolaire soumise (après application sur la peau ou les cheveux) à des contacts avec de l'eau. On caractérise la protection solaire attachée à une composition donnée en lui affectant un indice de protection (ou IP) qui s'exprime mathématiquement par le rapport du temps d'irradiation nécessaire pour atteindre le seuil érythématogène avec le filtre UV au temps nécessaire pour atteindre le seuil érythématogène sans filtre UV.

L'invention a donc pour objet une composition comprenant une dispersion aqueuse de vésicules lipidiques encapsulant un composé filtrant les radiations UV et comportant au moins une fonction acide, libre ou au moins partiellement neutralisée, caractérisée par le fait que les vésicules lipidiques ont une membrane lipidique formée à partir d'au moins un lipide amphiphile non-ionique (A) ou d'au moins un lipide amphiphile ionique (B) hydrocarboné saturé ayant un indice d'iode inférieur à 10 et d'au moins un lipide amphiphile ionique totalement neutralisé (C).

Les compositions conformes à l'invention sont plus particulièrement des dispersions huile-dans-eau dans lesquelles les vésicules lipidiques agissent comme agent dispersant de l'huile dans la phase aqueuse continue de la dispersion.

Les vésicules lipidiques conformes à l'invention sont plus particulièrement des vésicules à coeur aqueux (encapsulant une phase aqueuse).

On entend par vésicules lipidiques à coeur aqueux, des particules formées d'une membrane constituée par un ou plusieurs feuillets concentriques, ces feuillets comportant une ou plusieurs couches bimoléculaires de lipides amphiphiles encapsulant une phase aqueuse. La phase aqueuse peut contenir des substances actives hydrosolubles et les couches bimoléculaires de lipides amphiphiles peuvent contenir des substances actives lipophiles.

Les vésicules à coeur aqueux susmentionnées peuvent être préparées par de nombreux procédés. Selon un premier procédé, qui est par exemple décrit par BANGHAM et al. (J. Mol. Bio., 13, 1965 - pages 238 à 262), on dissout la phase lipidique sur les parois d'un flacon par évaporation du solvant, on introduit la phase à encapsuler sur le film lipidique et on agite le mélange mécaniquement jusqu'à obtention de la dispersion de vésicules à la taille désirée ; on obtient ainsi une dispersion de vésicules encapsulant une phase aqueuse, la phase aqueuse encapsulée et la phase aqueuse de dispersion étant identiques. Selon un second procédé dit "par cofusion des lipides", décrit par exemple dans FR-A-2315991, on prépare la phase lipidique par mélange du (des) lipide(s) amphiphile(s) et des éventuels additifs, à une température où le mélange est fondu, si le mélange n'est pas liquide à température ambiante ; on forme une phase lamellaire, par introduction de la phase aqueuse à encapsuler ; puis on disperse la phase lamellaire sous forme de vésicules, à l'aide d'un ultra-disperseur, d'un homogénéiseur ou d'ultrasons, dans une phase aqueuse de dispersion. Dans une variante de ce procédé, la formation de la phase lamellaire ne constitue pas un stade séparé du procédé. Les vésicules obtenues par ces deux procédés sont généralement de type "multifeuillet". Pour obtenir des vésicules de type "monofeuillet", on peut utiliser l'enseignement de FR-A-2543018.

Les lipides amphiphiles non-ioniques (A) formant la membrane des vésicules de l'invention sont de préférence choisis dans le groupe formé par :

(i) les esters et/ou les éthers de polyol et d'acide gras, polyoxyéthylénés ou non ;
(ii) les esters et/ou les éthers d'acide gras d'$\alpha$-butylglycoside.

Les esters ou les éthers de polyol et d'acide gras sont de préférence choisis parmi les mélanges d'esters ou les mélanges d'éthers d'au moins un polyol choisi dans le groupe formé par le polyéthylèneglycol comportant de 1 à 60 unités oxyde d'éthylène, le sorbitane, le sorbitane portant 2 à 60 unités d'oxyde d'éthylène, le glycérol portant 2 à 30 unités d'oxyde d'éthylène, les polyglycérols comportant 2 à 15 unités glycérol, les sucroses, les glucoses portant 2 à 30 unités oxyde d'éthylène, et au moins un acide gras comportant une chaîne alkyle en $C_5$-$C_{22}$, saturée ou non saturée, linéaire ou ramifiée, le nombre de chaînes alkyle par groupe polyol étant compris entre 1 et 10.

Les esters de polyol et d'acides gras en $C_5$-$C_{22}$ particulièrement préférés sont ceux répondant à la formule :

$$R\ CO[O\ CH_2—CHOH—CH_2]_nOH$$

où n est une valeur statistique et qui peut contenir des proportions diverses d'esters pour lesquels n = 1, n = 2, n = 3, n = 4, etc; c'est aussi le cas des esters comportant plusieurs chaînes alkyle dans leur partie lipophile, tels que les cocoates, qui contiennent des chaînes alkyles en $C_5$-$C_{22}$ ou les isostéarates où les chaînes alkyle sont en $C_{17}$ sont un mélange complexe de formes isomères ; c'est également le cas des produits constitués par des mélanges de mono-, di-, tri- ou polyesters d'un même polyol.

Parmi les produits commerciaux utilisables selon l'invention et ayant la structure d'un mélange d'esters de polyol et d'acide gras en $C_5$-$C_{22}$ tel que défini ci-dessus, on peut citer :

- les esters partiels de sorbitane (ou anhydride de sorbitol) et d'acide gras, vendus sous les dénominations commerciales "SPAN 20, 40, 60 et 80" par la Société "ICI" ;
- l'isostéarate de sorbitane, vendu sous la dénomination commerciale "SI 10 R NIKKOL" par la Société "NIKKO" ;
- le stéarate de sorbitane portant 4 unités oxyde d'éthylène, vendu sous la dénomination "TWEEN 61" par la Société "ICI" ;
- le stéarate de polyéthylèneglycol à 8 unités oxyde d'éthylène vendu sous le nom "MYR J 45" par "ICI" ;
- le monostéarate du polyéthylène glycol de formule

$$OHCH_2(CH_2OCH_2)_nCH_2OH$$

formule dans laquelle n est égal à 4, vendu sous la dénomination "MYS 4" par la Société "NIKKO" ;

- le stéarate de polyéthylèneglycol de poids moléculaire 400, qualité chimique ou qualité produite par biotechnologie, vendu par la Société "UNICHEMA" ;
- le stéarate de diglycéryle portant 4 unités d'oxyde d'éthylène, vendu sous la dénomination "HOSTACERINE DGS" par la Société "HOECHST" ;
- le stéarate de tétraglycérol, vendu sous la dénomination "TETRAGLYN 1S" par la Société "NIKKO" ;
- l'isostéarate de diglycéryle, vendu par la Société "SOLVAY" ;
- le distéarate de diglycéryle, vendu sous la dénomination "EMALEX DSG 2" par la Société "NIHON" ;
- les mono-, di- et tri-palmitostéarate de sucrose, vendu sous les dénominations "F50, F70, F110 et F160 CRODESTA" par la Société "CRODA" ;
- le mélange de mono- et di-palmitostéarate de sucrose, vendu sous la dénomination "GRILLOTEN PSE 141 G" par la Société "GRILLO" ;
- le mélange de stéarate de sucrose et de cocoate de sucrose, vendu sous la dénomination "ARLATONE 2121" par la Société "ICI";
- le distéarate de méthylglucose portant 20 unités oxyde d'éthylène, vendu sous la dénomination "GLUCAM E20 DISTEARATE" par la Société "AMERCHOL".

Les esters et les éthers d'acide gras d'α-butylglucoside utilisés selon l'invention sont de préférence, soit des mélanges d'esters et/ou des mélanges d'éthers de différents acides gras d'α-butylglucoside dont les différentes chaînes grasses comportent, l'une par rapport à l'autre, un nombre d'atomes de carbone voisin (par exemple différent de 1 ou 2) soit des mélanges de mono-, di- tri- ou polyesters et/ou des mélanges de mono-, di-tri-polyéthers d'un même acide gras d'α-butylglucoside.

Les esters et les éthers d'acide gras d'α-butylglucoside utilisé(s) selon l'invention comporte(nt) de préférence une chaîne grasse ayant de 8 à 24 atomes de carbone, plus préférentiellement de 12 à 22 atomes de carbone et plus particulièrement de 14 à 18 atomes de carbone.

On peut citer par exemple, les esters et les éthers d'acide laurique ($C_{12}$), myristique ($C_{14}$), palmitique ($C_{16}$), stéarique ($C_{18}$), béhénique ($C_{22}$) d'α-butylglucoside.

On utilise plus particulièrement un mélange de mono- et de diester d'acide palmitique d'α-butylglucoside obtenu selon le procédé de fabrication enzymatique décrit dans les vésicules lipidiques conformes à l'invention.

Les esters et les éthers d'acide gras d'α-butylglucoside conformes à l'invention peuvent être préparés à partir d'α-butylglucoside obtenu selon le procédé de fabrication enzymatique décrit dans la demande de brevet FR-A-2680373 qui consiste à mettre en contact le butanol avec de l'amidon, des maltodextrines ou du maltose en présence d'une préparation enzymatique purifiée présentant une activité d'α-transglucosylation. Les esters et les éthers d'acide gras d'α-butylglucoside peuvent être synthétisés en faisant réagir l'acide gras ou le mélange d'acide gras correspondants avec l'α-butylglucoside selon des procédés classiques.

Les lipides amphiphiles ioniques hydrocarbonés saturés (B) (indice d'iode inférieur à 10) formant la membrane des vésicules de l'invention sont choisis de préférence parmi les phospholipides de synthèse tels que la dipalmitoylphosphatidylcholine ou les phospholipides modifiés par voie chimique ou enzymatique tels que la lécithine hydrogénée.

Les lipides amphiphiles ioniques totalement neutralisés (C) sont choisis de préférence parmi ceux ayant des fonctions acides totalement neutralisés et plus particulièrement dans le groupe constitué par:

(i) les acylglutamates disodiques ou dipotassiques ;
(ii) les sels alcalins du dicétylphosphate et du dimyrystylphosphate en particulier les sels de Na et K ;
(iii) les phospholipides ;
(iv) les dérivés alkylsulfoniques de formule :

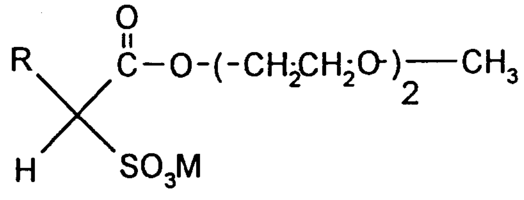

formule dans laquelle R représente des radicaux en $C_{12}$-$C_{22}$, en particulier les radicaux $C_{16}H_{33}$ et $C_{18}H_{37}$, pris en mélange ou séparément et M est un métal alcalin, de préférence le sodium ;

(v) les dérivés d'acide salicylique tels que décrits et préparés dans la demande de brevet EP-A-585170 de formule (1) :

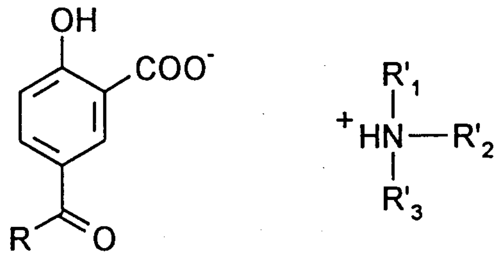

(1)

dans laquelle R représente un radical alkyle linéaire ou ramifié en $C_{11}$-$C_{17}$ ; R'$_1$, R'$_2$, R'$_3$, identiques ou différents, représentent un radical alkyle ou hydroxyalkyle en $C_1$-$C_{18}$, un des radicaux R'$_1$, R'$_2$, R'$_3$ pouvant être un radical benzyle.

(vi) les sels alcalins du cholestérol-sulfate, en particulier le sel de Na ;

(vii) les sels alcalins du cholestérol-phosphate, en particulier le sel de Na ;

(viii) les sels alcalins de l'acide phosphatidique, en particulier le sel de Na.

Les lipides amphiphiles ioniques totalement neutralisés (C) particulièrement préférés sont choisis dans le groupe constitué par les acylglutamates disodiques tels que le sel disodique de l'acide N-stéaroyl glutamique commercialisé sous la dénomination "Acylglutamate HS 21" par la Société Ajinomoto et les dérivés salicyliques de formule (1) tels que le n-dodecanoyl-5 salicylate de N,N-diméthyl N-(hydroxy-2 éthyl) ammonium.

Selon un mode préférentiel de l'invention, le rapport en poids entre la quantité de lipide amphiphile non-ionique (A) ou de lipide amphiphile ionique hydrocarboné saturé (B) et la quantité de lipide amphiphile ionique totalement neutralisé (c) étant compris entre 50/1 et 50/25 et le rapport en poids entre la quantité de phase lipidique et la quantité de phase aqueuse de la dispersion étant compris entre 1/1000 et 300/1000.

Dans la composition selon l'invention, les vésicules à coeur aqueux ont de préférence un diamètre moyen allant de 10 à 5 000 nm.

Les vésicules des compositions selon l'invention peuvent contenir en plus un ou plusieurs composé(s) actif(s) ayant une activité cosmétique et/ou dermopharmaceutique, qui, selon leurs caractéristiques de solubilité, peuvent avoir différentes localisations. Si les actifs sont hydrosolubles, on les introduit, de préférence, dans la phase aqueuse encapsulée des vésicules à coeur aqueux. Si les actifs sont liposolubles, on les introduit, de préférence, dans la phase lipidique constituant la membrane.

Si les actifs sont amphiphiles, ils se répartissent entre la phase lipidique et la phase aqueuse encapsulée des vésicules à coeur aqueux avec un coefficient de partage qui varie selon la nature de l'actif amphiphile.

On peut, de façon connue, incorporer dans la phase lipidique constituant la membrane lipidique des vésicules à coeur aqueux de l'invention, au moins un additif qui a pour fonction principale de diminuer la perméabilité des vésicules, de prévenir leur floculation et leur fusion et d'augmenter le taux d'encapsulation.

Selon un mode préférentiel de l'invention, on peut ajouter à la phase lipidique au moins un additif choisi, de préférence, dans le groupe formé par:

- les stérols et notamment les phytostérols et le cholestérol,
- les alcools et diols à longue chaîne,
- les amines à longue chaîne et leurs dérivés ammonium quaternaire.

Ces additifs peuvent éventuellement avoir une activité cosmétique et/ou dermopharmaceutique. C'est, par exemple, le cas du cholestérol.

Les composés filtres UV ayant au moins une fonction acide, libre ou au moins partiellement neutralisée, selon la présente invention sont choisis de préférence parmi les filtres hydrosolubles ayant au moins une foncion acide sulfonique $SO_3H$.

Comme exemple de filtres acides contenant au moins un groupe $SO_3H$, on peut citer les dérivés sulfoniques du 3-benzylidène 2-bornanone et notamment ceux de formules (I), (II), (III), (IV), et (V) suivantes :

*Formule (I) :*

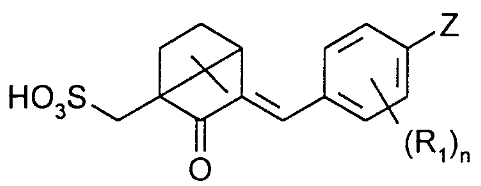

(I)

dans laquelle :

- Z désigne un groupement

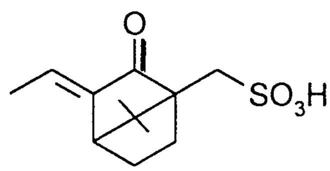

- n est égal à 0 ou est un nombre entier compris entre 1 et 4($0 \leq n \leq 4$)
- $R_1$, représente un ou plusieurs radicaux alkyle ou alkoxy, identiques ou différents, linéaires ou ramifiés, contenant de 1 à 4 atomes de carbone environ

Un composé de formule I particulièrement préféré est celui correspondant à n = 0 : l'acide benzène 1,4 [di(3-méthylidènecampho 10-sulfonique)].

*Formule (II) :*

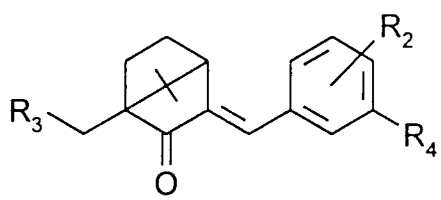

(II)

dans laquelle :

- $R_2$ désigne un atome d'hydrogène, un atome d'halogène, un radical alkyle contenant de 1 à 4 atomes de carbone environ ou un radical $-SO_3H$.
- $R_3$ et $R_4$ désignent un atome d'hydrogène ou un radical $-SO_3H$ au moins un des radicaux $R_2$, $R_3$ ou $R_4$ désignant le radical $-SO_3H$, $R_2$ et $R_4$ ne pouvant désigner simultanément un radical-$SO_3H$.

On peut citer comme exemples particuliers les composés suivants de formule II dans laquelle :

- $R_2$ désigne le radical $-SO_3H$ en position para du benzylidènecamphre et $R_3$ et $R_4$ désignent chacun un atome d'hydrogène, c'est-à-dire l'acide 4-(3-méthylidènecamphre) benzène sulfonique.
- $R_2$ et $R_4$ désignent chacun un atome d'hydrogène et $R_3$ désigne un radical $-SO_3H$, c'est-à-dire l'acide 3-benzylidène campho-10-sulfonique.

- $R_2$ désigne un radical méthyle en position para du benzylidènecamphre, $R_4$ un radical $-SO_3H$ et $R_3$ un atome d'hydrogène, c'est-à-dire l'acide 2-méthyl 5-(3-méthylidènecamphre) benzène sulfonique.
- $R_2$ désigne un atome de chlore en position para du benzylidènecamphre, $R_4$ un radical - $SO_3H$ et $R_3$ un atome d'hydrogène, c'est-à-dire l'acide 2-chloro 5-(3-méthylidènecamphre) benzène sulfonique.
- $R_2$ désigne un radical méthyle en position para du benzylidènecamphre, $R_4$ désigne un atome d'hydrogène et $R_3$ désigne un radical $-SO_3H$, c'est-à-dire l'acide 3-(4-méthyl) benzylidène campho 10-sulfonique.

*Formule (III) :*

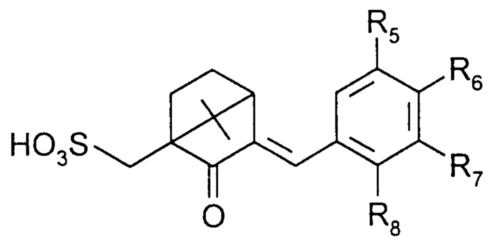

(III)

dans laquelle:

- $R_5$ et $R_7$ désignent un atome d'hydrogène, un radical hydroxyle, un radical alkyle ou alcoxy, linéaire ou ramifié, contenant de 1 à 8 atomes de carbone environ, l'un au moins des radicaux $R_5$ et $R_7$ représentant un radical hydroxyle, alkyle ou alcoxy,
- $R_6$ et $R_8$ désignent un atome d'hydrogène, un radical hydroxyle, l'un au moins des radicaux $R_6$ et $R_8$ désignent le radical hydroxyle, sous réserve que lorsque $R_5$ et $R_8$ désignent un atome d'hydrogène et que $R_6$ désigne un radical hydroxyle, $R_7$ ne désigne pas un radical alcoxy ou un atome d'hydrogène.

On peut citer comme exemples particuliers les composés suivants de formule (III) dans laquelle :

- $R_5$ est un radical méthyle, $R_6$ un atome d'hydrogène, $R_7$ un radical tertiobutyle, $R_8$ un radical hydroxyle, c'est-à-dire l'acide (3-t-butyl 2-hydroxy 5-méthyl) benzylidène campho-10-sulfonique.
  - $R_5$ est un radical méthoxy, $R_6$ un atome d'hydrogène, $R_7$ un radical tertiobutyle, $R_8$ un radical hydroxyle, c'est-à-dire l'acide (3-t-butyl 2-hydroxy 5-méthoxy) benzylidène campho-10-sulfonique.
  - $R_5$ et $R_7$ désignent chacun un radical tertiobutyle, $R_6$ un radical hydroxyle, $R_8$ un atome d'hydrogène, c'est-à-dire l'acide (3,5-diterbutyl 4-hydroxy) benzylidène campho-10-sulfonique.

*Formule (IV) :*

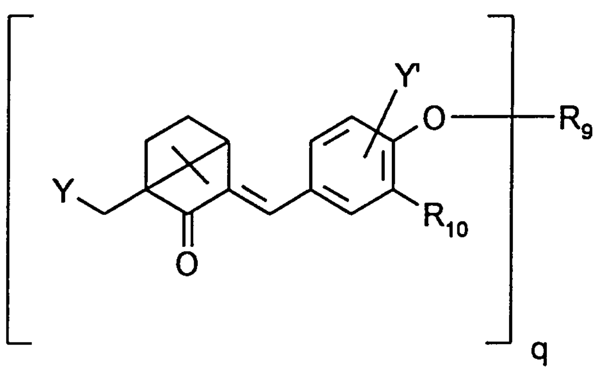

(IV)

dans laquelle :

- $R_9$ désigne un atome d'hydrogène, un radical alkyle, linéaire ou ramifié, contenant de 1 à 18 atomes de carbone environ, un radical alcényle, linéaire ou ramifié, contenant de 3 à 18 atomes de carbone environ, un groupement

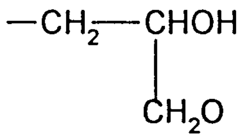

$-(CH_2CH_2O)_n-H$, ou $-CH_2-CHOH-CH_3$,

ou encore un radical divalent : $-(CH_2)_m-$ ou $-CH_2-CHOH-CH_2-$
n étant un nombre entier compris entre 1 et 6 ($1 \leq n \leq 6$) et m un nombre entier compris entre 1 et 10 ($1 \leq m \leq 10$),

- $R_{10}$ désigne un atome d'hydrogène, un radical alcoxy contenant de 1 à 4 atomes de carbone environ ou un radical divalent -O- relié au radical $R_9$ lorsque celui-ci est divalent lui aussi,
- q désigne un nombre entier égal à 1 ou 2, étant entendu que si q est égal à 2, $R_9$ doit désigner un radical divalent,
- Y et Y' désignent un atome d'hydrogène ou un radical $-SO_3H$, au moins un de ces radicaux Y ou Y' est différent de l'hydrogène.

On peut citer comme exemples particuliers les composés suivants de formule (IV) dans laquelle :

- q est égal à 1, Y et R10 désignent chacun un atome d'hydrogène, $R_9$ désigne un radical méthyle, Y' en position 3 désigne un radical $-SO_3H$, c'est-à-dire l'acide 2-méthoxy 5-(3-méthylidènecamphre) benzène sulfonique.
- q est égal à 1, Y désigne un radical $-SO_3H$, Y' un atome d'hydrogène, $R_{10}$ un radical divalent -O- relié à $R_9$ désignant un radical méthylène, c'est-à-dire l'acide 3-(4,5-méthylènedioxy) benzylidène campho-10-sulfonique.
- q est égal à 1, Y désigne un radical $-SO_3H$, Y' et $R_{10}$ désignent tous deux un atome d'hydrogène, $R_9$ désigne un radical méthyle, c'est-à-dire l'acide 3-(4-méthoxy) benzylidène campho-10-sulfonique.
- q est égal à 1, Y désigne un radical $-SO_3H$, Y' un atome d'hydrogène ; $R_9$ désigne un radical méthyle, $R_{10}$ désigne un radical méthoxy, c'est-à-dire l'acide 3-(4,5-diméthoxy) benzylidène campho-10-sulfonique.
- q est égal à 1, Y désigne un radical $-SO_3H$, Y' et $R_{10}$ désignent tous deux un atome d'hydrogène ; $R_9$ un radical n butyle, c'est-à-dire l'acide 3-(4-n-butoxy) benzylidène campho-10-sulfonique.
- q est égal à 1, Y désigne un radical $-SO_3H$, Y' un atome d'hydrogène; $R_9$ désigne un radical n butyle, $R_{10}$ un radical méthoxy, c'est-à-dire l'acide 3-(4-n-butoxy 5-méthoxy) benzylidène campho-10-sulfonique.

*Formule (V) :*

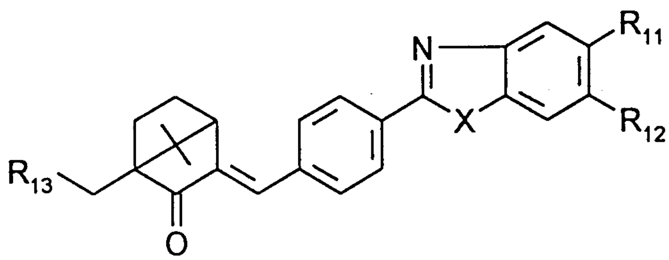

(V)

dans laquelle :

- $R_{11}$ désigne un atome d'hydrogène, un radical alkyle ou alcoxy, linéaire ou ramifié, contenant de 1 à 6 atomes de carbone environ ou un radical $-SO_3H$,
- $R_{12}$ désigne un atome d'hydrogène, un radical alkyle ou alcoxy, linéaire ou ramifié, contenant de 1 à 6 atomes de carbone environ,
- $R_{13}$ désigne un atome d'hydrogène ou un radical $-SO_3H$,
- l'un au moins des radicaux $R_{11}$ et $R_{13}$ désignant un radical $-SO_3H$,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer comme exemple particulier de formule (V) : le composé dans lequel X désigne un radical -NH-, $R_{11}$ désigne un radical $-SO_3H$, $R_{12}$ et $R_{13}$ désignent tous deux un atome d'hydrogène, c'est-à-dire l'acide 2-[4-(camphométhylidène) phényl] benzimidazole-5-sulfonique.

Les composés de structures (I), (II), (III), (IV), (V) sont respectivement décrits dans le brevet US 4.585.597 et les brevets FR 2.236.515, 2.282.426, 2.645.148, 2.430.938, 2.592.380.

Le filtre à groupement sulfonique peut également être un dérivé sulfonique de benzophénone de formule (VI) :

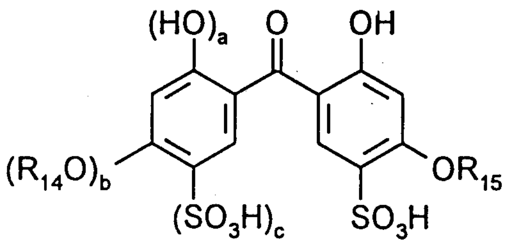

(VI)

dans laquelle :

- $R_{14}$ et $R_{15}$, identiques ou différents, désignent soit un atome d'hydrogène soit un radical alkyle, linéaire ou ramifié, contenant de 1 à 8 atomes de carbone environ.
- a, b et c, identiques ou différents, sont égaux à 0 ou 1.

On peut citer comme exemple particulier de formule (VI) : l'acide 2-hydroxy 4-méthoxybenzophénone 5-sulfonique (composé de formule VI dans laquelle a, b, et c sont égaux à zéro, et $R_{15}$ désigne un radical méthyle).

Le filtre à groupement sulfonique peut encore être un dérivé sulfonique de benzimidazole de formule :

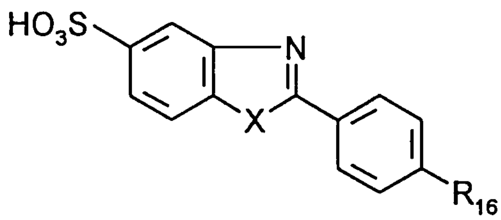

(VII)

dans laquelle :

- X désigne un atome d'oxygène ou un radical -NH-
- $R_{16}$ désigne un atome d'hydrogène, un radical alkyle ou alcoxy, linéaire ou ramifié, contenant de 1 à 8 atomes de carbone environ ou un groupement de formule

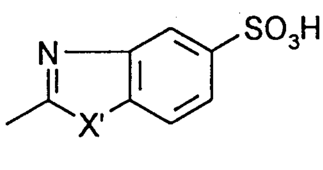

(VIII)

dans laquelle X' représente un atome d'oxygène ou un radical -NH-.

On peut citer comme exemples particuliers les composés suivants de formule (VII) dans laquelle :

- X désigne le radical -NH- et $R_{16}$ désigne un atome d'hydrogène : l'acide 2-phénylbenzimidazole 5-sulfonique.
- X désigne le radical -NH-, $R_{16}$ désigne le groupement de formule (VIII) dans lequel X' désigne le radical -NH- : l'acide benzène 1,4 -di(benzimidazol -2 yl-5-sulfonique).
- X désigne un atome d'oxygène, $R_{16}$ désigne le groupement de formule (VIII) dans lequel X' désigne un atome d'oxygène : l'acide benzène 1,4-di (benzoxazol -2 yl -5-sulfonique).

Les composés de formule VI et VII sont des composés connus pouvant être préparés selon des méthodes classiques décrites dans l'art antérieur.

Les composés filtres à fonction acide, sont présents dans les compositions de l'invention dans des concentrations allant préférentiellement de 0,1 à 10 % en poids en matière active par rapport au poids total de la composition et plus particulièrement de 0,5 à 5 % en poids en matière active.

La phase aqueuse continue de la composition conforme à l'invention contient de préférence une huile dispersée dans celle-ci par les vésicules lipidiques.

L'huile, peut notamment être choisie dans le groupe formé par :

- les huiles animales ou végétales formées par des esters d'acide gras et de polyols, en particulier les triglycérides liquides, par exemple les huiles de tournesol, de maïs, de soja, de courge, de pépins de raisin, de jojoba, de sésame, de noisette, les huiles de poisson, le tricaprocaprylate de glycérol, ou leurs huiles végétales ou animales de formule $R_9COOR_{10}$, formule dans laquelle $R_9$ représente le reste d'un acide gras supérieur comportant de 7 à 19 atomes de carbone et $R_{10}$ représente une chaîne hydrocarbonée ramifiée contenant de 3 à 20 atomes de carbone, par exemple l'huile de Purcellin ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote ;
- des hydrocarbures, tels que l'hexadécane et l'huile de paraffine ;
- des carbures halogénés, notamment des fluorocarbures tels que des fluoroamines par exemple la perfluorotributylamine, des hydrocarbures fluorés, par exemple le perfluorodécahydronaphtalène, des fluoroesters et des fluoroéthers ;
- des silicones, par exemple les polysiloxanes, les polydiméthylsiloxanes et les fluorosilicones ;
- des esters d'acide minéral et d'un alcool ; et
- des éthers et des polyéthers.

La phase aqueuse continue de la composition selon l'invention peut aussi également contenir des actifs cosmétiques et/ou dermopharmaceutiques, hydrosolubles. L'huile dispersée par les vésicules lipidiques peut éventuellement contenir un actif liposoluble.

La phase aqueuse continue de dispersion peut aussi contenir des adjuvants n'ayant ni activité cosmétique ni activité dermopharmaceutique propre, mais utilisés pour la formulation de la dispersion sous forme de lotion, crème, lait ou sérum. Ces adjuvants sont, en particulier, pris dans le groupe formé par les gélifiants, les conservateurs, les colorants, les opacifiants et les parfums. Parmi les gélifiants utilisables, on peut citer les dérivés d'algues tels que le satiagum, des gommes naturelles, telles que l'adragante, et des polymères synthétiques, en particulier les mélanges d'acides polycarboxyvinyliques commercialisés sous la dénomination "HOSTACERIN PN 73" par la Société "HOECHST" ou sous le nom "CARBOPOL" par "GOODRICH".

Un autre objet de l'invention consiste en des compositions à usage topique. Elles sont caractérisées par le fait qu'elles sont constituées par les compositions telles que définies ci-dessus.

Un autre objet de l'invention consiste en l'utilisation des compositions telles que définies précédemment pour le soin de la peau et/ou du cuir chevelu et/ou des cheveux et/ou des cils ou des sourcils et/ou comme base de produits pour le maquillage.

Un autre objet de l'invention consiste en l'utilisation des compositions telles que définies précédemment comme base de produits pour le maquillage.

Les produits cosmétiques ou dermopharmaceutiques obtenus à partir des compositions de l'invention peuvent se présenter sous forme de dispersion huile-dans-eau plus ou moins épaissie, de gel, de crème, de lait ou de sérum.

Les compositions cosmétiques de l'invention peuvent être utilisées comme composition protectrice de l'épiderme humain, des cheveux, des cils ou des sourcils contre les rayons ultraviolets, comme produit antisolaire ou comme produit de maquillage.

Lorsque les compositions cosmétiques selon l'invention sont utilisées pour la protection des cheveux et du cuir chevelu, elle peuvent se présenter sous forme de shampooing, de lotion, de gel et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque les compositions cosmétiques ou dermatologiques sont utilisées pour la protection de la peau ou des cils, des sourcils, elles peuvent se présenter sous forme de crème de traitement de l'épiderme, de fond de teint, de mascara ou de ligneur encore appelé "eye liner".

Un autre objet de la présente invention est un procédé de traitement non-thérapeutique de la peau, du cuir chevelu des cheveux, des cils ou des sourcils destiné à les protéger contre les effets des rayons UV consistant à appliquer sur

ceux-ci une quantité efficace d'une composition cosmétique telle que définie ci-dessus.

Les exemples ci-après, donnés à titre purement illustratif et non limitatif permettront de mieux comprendre l'invention.

Dans tous les exemples donnés ci-après les dispersions de vésicules sont préparées par le procédé dit "par cofusion des lipides" dans lequel :

- dans une première étape, on prépare la phase lipidique par mélange sous forme liquide de différents lipides amphiphiles la composant, et éventuellement associés à des additifs ou des actifs liposolubles, et on met la phase lipidique obtenue en présence d'une phase aqueuse contenant éventuellement des actifs hydrosolubles, de façon à obtenir une phase lamellaire,
- dans une deuxième étape, on ajoute à la phase lamellaire hydratée obtenue une phase aqueuse de dispersion contenant éventuellement une huile et différents additifs,
- et dans une troisième étape, on soumet le mélange à une agitation énergique dans un homogénéiseur pour obtenir des vésicules dispersées dans une phase aqueuse de dispersion.

| **EXEMPLE 1: Sérum pour le visage**<br>*Première phase :* | |
|---|---|
| - Stéarate de polyéthylèneglycol de poids moléculaire 400 (A), produit par biotechnologie vendu par la Société "Unichema" | 0,95 % |
| - Cholesterol | 0,95 % |
| - Sel disodique de l'acide N-stéaroyl glutamique (C) commercialisé sous la dénomination "Acylglutamate HS 21" par la Société Ajinomoto | 0,1 % |
| - Acide benzène 1,4 (di(3-méthylidènecampho-10-sulfonique)] (filtre UV acide) | 2,0 % |
| - Triéthanolamine pour neutralisation du filtre UV acide | 0,4 % |
| - Glycérine | 3,00 % |
| - Conservateurs | 0,3 % |
| - Hydrolysat lactique commercialisé sous le nom LACTOLAN LS par les Laboratoires Sérobiologiques de Nancy | 5,0 % |
| - Solution aqueuse de superoxyde dismutase à 5,000 U/ml commercialisé par la Société Pentapham | 1,0 % |
| *Deuxième phase* : | |
| - Mélange d'acide polycarboxyvinylique commercialisé sous la dénomination "Carbopol" (gélifiant) par la Société Goodrich | 0,20 % |
| - L-lysine monohydrate | qs pH 6,5 |
| - Eau déminéralisée | qsp 100 % |

| **<u>EXEMPLE 2</u> : Crème de jour pour le visage**<br>*Première phase* : | | |
|---|---|---|
| - Stéarate de sorbitane à 4 moles d'oxyde d'éthylène vendu sous le nom TWEEN 61 par ICI (A) | | 3,8 % |
| - Dodecanoyl-5 salicylate de N,N-diméthyl N-(hydroxy-2 éthyl) ammonium (C) | | 0,4 % |
| - Cholestérol | | 3,8 % |
| - Acide benzène 1,4 (di(3-méthylidènecampho-10-sulfonique)] (filtre UV acide) | | 5,00 % |
| - Triéthanolamine pour neutralisation du filtre UV acide | | 1,0 % |
| - Acétate de tocophérol | | 0,5 % |
| - Glycérine | | 5,0 % |
| - Eau déminéralisée | qsp | 50 % |
| *Deuxième phase* : | | |
| - Huile de macadamia | | 20,0 % |
| - Parfum | | 0,2 % |
| - Mélange d'acides polycarboxyvinyliques commercialisé sous la dénomination "Carbopol" (gélifiant) par la Société Goodrich | | 0,42 % |
| - Conservateurs | | 0,3 % |
| - Triéthanolamine | qsp | pH = 6 |
| - Eau déminéralisée | qsp | 100 % |

| | | |
|---|---|---|
| **<u>EXEMPLE 3</u> :** Fluide de jour pour le visage<br>*Première phase* : | | |
| - Stéarate de polyéthylèneglycol de poids moléculaire 400 (A), produit par biotechnologie vendu par la Société Unichema | | 0,9 % |
| - Dodecanoyl-5 salicylate de N,N-diméthyl N-(hydroxy-2 éthyl) ammonium (C) | | 0,1 % |
| - Acide benzène 1,4 (di(3-méthylidènecampho-10-sulfonique)] (filtre UV acide) | | 3,0 % |
| - Triéthanolamine pour neutralisation du filtre UV acide | | 1,0 % |
| - Glycérine | | 3,0 % |
| - Eau déminéralisée | qsp | 65 % |
| *Deuxième phase* : | | |
| - Acétate de tocophérol | | 1,0 % |
| - Huile de jojoba | | 5,0 % |
| - Huile de silicone volatile | | 4,0 % |
| - parfum | | 0,3 % |
| - Mélange d'acides polycarboxyvinyliques commercialisé sous la dénomination "Carbopol" (gélifiant) par la Société Goodrich | | 0,1 % |
| - Triéthanolamine | qsp | pH = 6 |
| - Eau déminéralisée | qsp | 100 % |

| **EXEMPLE 4: Crème de jour pour le visage** *Première phase:* | | |
|---|---|---|
| - Distéarate de diglycéryle (A) vendu sous le nom EMALEX PSGA par la société NIHON EMULSION | | 3,6 % |
| - Sel disodique de l'acide N-stéaroyl glutamique (C) commercialisé sous la dénomination "Acylglutamate HS 21" par la Société Ajinomoto | | 0,8 % |
| - Cholestérol | | 3,6 % |
| - Acide benzène 1,4 (di(3-méthylidènecampho-10-sulfonique)] (filtre UV acide) | | 7,0% |
| - Triéthanolamine pour neutralisation du filtre UV acide | | 1,4 % |
| - Acétate de tocophérol | | 0,5 % |
| - Glycérine | | 5,0 % |
| - Eau déminéralisée | qsp | 50 % |
| *Deuxième phase:* | | |
| - Huile d'amande douce | | 4,0 % |
| - Huile de pépins de raisin | | 8,0 % |
| - Huile de tournesol | | 8,0 % |
| - Huile de silicone volatile | | 3,0 % |
| - Parfum | | 0,2 % |
| - Mélange d'acides polycarboxyvinyliques commercialisé sous la dénomination "Carbopol " (gélifiant) par la Société GOODRICH | | 0,42 % |
| - Triéthanolamine | qsp | pH = 6 |
| - Eau déminéralisée | qsp | 100 % |

**Revendications**

1. Composition comprenant une dispersion aqueuse de vésicules lipidiques encapsulant un composé filtrant les radiations UV et comportant au moins une fonction acide, libre ou au moins partiellement neutralisée, caractérisée par le fait que les vésicules lipidiques ont une membrane lipidique formée à partir d'au moins un lipide amphiphile non-ionique (A) ou d'au moins un lipide amphiphile ionique (B) hydrocarboné ayant un indice d'iode inférieur à 10 et d'au moins un lipide amphiphile ionique totalement neutralisé (C).

2. Composition selon la revendication 1, caractérisée en ce qu'elle est une dispersion huile-dans-eau dans laquelle les vésicules lipidiques agissent comme agent dispersant de l'huile dans la phase aqueuse continue de la dispersion.

3. Composition selon la revendication 1 ou 2, caractérisée par le fait que les vésicules sont des vésicules lipidiques à coeur aqueux (encapsulant une phase aqueuse).

4. Composition selon l'une quelconque des revendications 1 à 3, caractérisée par le fait que les lipides amphiphiles non-ioniques (A) formant la membrane des vésicules sont choisis dans le groupe formé par :

   (i) les esters et/ou les éthers de polyol et d'acide gras, polyoxyéthylénés ou non ;
   (ii) les esters et/ou les éthers d'acide gras d'$\alpha$-butylglycoside.

5. Composition selon la revendication 4, caractérisée par le fait que les lipides amphiphiles non-ioniques (A) sont choisis parmi les mélanges d'esters et/ou les mélanges d'éthers d'au moins un polyol choisi dans le groupe formé par le polyéthylèneglycol comportant de 1 à 60 unités oxyde d'éthylène, le sorbitane, le sorbitane portant 2 à 60 unités d'oxyde d'éthylène, le glycérol portant 2 à 30 unités d'oxyde d'éthylène, les polyglycérols comportant 2 à 15

unités glycérol, les sucroses, les glucoses portant 2 à 30 unités oxyde d'éthylène, et au moins un acide gras comportant une chaîne alkyle en $C_5$-$C_{22}$, saturée ou non saturée, linéaire ou ramifiée, le nombre de chaînes alkyle par groupe polyol étant compris entre 1 et 10.

**6.** Composition selon la revendication 4, caractérisée par le fait que les lipides amphiphiles non-ioniques (A) sont choisis parmi les esters et/ou les éthers d'acide gras d'α-butylglucoside comportant une chaîne grasse ayant de 8 à 24 atomes de carbone, plus préférentiellement de 12 à 22 atomes de carbone et plus particulièrement de 14 à 18 atomes de carbone.

**7.** Composition selon la revendication 6, caractérisée par le fait que les lipides amphiphiles non-ioniques (A) sont choisis dans le groupe constitué par les esters et/ou les éthers d'acide laurique ($C_{12}$), myristique ($C_{14}$), palmitique ($C_{16}$), stéarique ($C_{18}$), béhénique ($c_{22}$) d'α-butylglucoside.

**8.** Composition selon la revendication 6 ou 7, caractérisée par le fait que les lipides amphiphiles non-ioniques (A) sont un mélange de mono- et de diester d'acide palmitique d'α-butylglucoside.

**9.** Composition selon l'une quelconque des revendications 6 à 8, caractérisée par le fait que les lipides amphiphiles non-ioniques (A) formant la membrane des vésicules sont choisis dans le groupe formé par des mélanges et/ou des mélanges d'éthers de différents acides gras d'α-butylglucoside dont les différentes chaînes grasses comportent, l'une par rapport à l'autre, un nombre d'atomes de carbone voisin et des mélanges de mono-, di- tri- ou polyesters et/ou de mono-, di- tri- ou polyéthers d'un même acide gras d'α-butylglucoside.

**10.** Composition selon l'une quelconque des revendications 1 à 9, caractérisée par le fait que les lipides amphiphiles ioniques hydrocarbonés saturés (B) formant la membrane des vésicules sont choisis dans le groupe formé par les phospholipides de synthèse ou les phospholipides modifiés par voie chimique ou enzymatique.

**11.** Composition selon l'une quelconque des revendications 1 à 10, caractérisée par le fait que les lipides amphiphiles ioniques totalement neutralisés (C) formant la membrane des vésicules sont choisis parmi ceux ayant des fonctions acides totalement neutralisés.

**12.** Composition selon la revendication 11, caractérisée par le fait que les lipides amphiphiles ioniques totalement neutralisés (C) formant la membrane des vésicules sont choisis dans le groupe constitué par :

(i) les acylglutamates disodiques ou dipotassiques ;
(ii) les sels alcalins du dicétylphosphate et du dimirystylphosphate ;
(iii) les phospholipides ;
(iv) les dérivés alkylsulfoniques de formule :

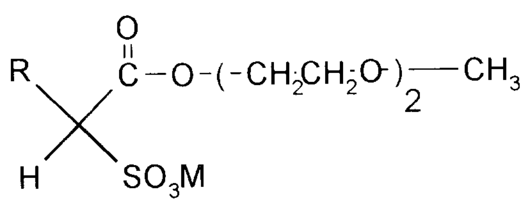

formule dans laquelle R représente des radicaux en $C_{12}$-$C_{22}$, en particulier les radicaux $C_{16}H_{33}$ et $C_{18}H_{37}$ , pris en mélange ou séparément et M est un métal alcalin ;
(v) les dérivés d'acide salicylique de formule (1) :

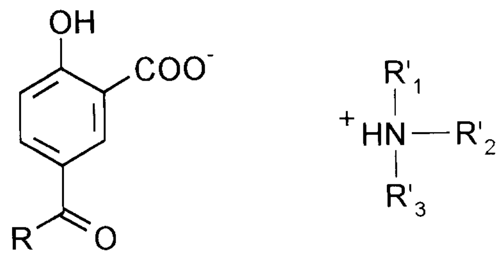

(1)

dans laquelle R représente un radical alkyle linéaire ou ramifié en $C_{11}$-$C_{17}$ ; R'$_1$, R'$_2$, R'$_3$ , identiques ou différents, représentent un radical alkyle ou hydroxyalkyle en $C_1$-$C_{18}$, un des radicaux R'$_1$, R'$_2$, R'$_3$ pouvant être un radical benzyle.

(vi) les sels alcalins du cholestérol-sulfate ;
(vii) les sels alcalins du cholestérol-phosphate ;
(viii) les sels alcalins de l'acide phosphatidique.

**13.** Composition selon la revendication 12, caractérisée par le fait que les lipides amphiphiles ioniques totalement neutralisés (C) formant la membrane des vésicules sont choisis dans le groupe constitué par le sel disodique de l'acide N-stéaroyl glutamique et le n-dodecanoyl-5 salicylate de N,N-diméthyl N-(hydroxy-2 éthyl) ammonium.

**14.** Composition selon l'une quelconque des revendications 1 à 13, caractérisée par le fait que le rapport en poids entre la quantité de lipide amphiphile non-ionique (A) ou de lipide amphiphile ionique hydrocarboné saturé (B) et la quantité de lipide amphiphile ionique totalement neutralisé (C) est compris entre 50/1 et 50/25 et le rapport en poids entre la quantité de phase lipidique et la quantité de phase aqueuse de la dispersion est compris entre 1/1000 et 300/1000.

**15.** Composition selon l'une quelconque des revendications 1 à 14, caractérisée par le fait que les vésicules contiennent en plus un ou plusieurs composé(s) actif(s) hydrosoluble(s), liposoluble(s) ou amphiphile(s) ayant une activité cosmétique et/ou dermopharmaceutique.

**16.** Composition selon l'une quelconque des revendications 1 à 15, caractérisée par le fait que les vésicules contiennent en plus au moins un additif ayant pour fonction principale de diminuer la perméabilité des vésicules, de prévenir leur floculation et leur fusion et d'augmenter le taux d'encapsulation.

**17.** Composition selon la revendication 16, caractérisée par le fait que les vésicules contiennent en plus au moins un additif choisi dans le groupe formé par :

- les stérols,
- les alcools et diols à longue chaîne,
- les amines à longue chaîne et leurs dérivés ammonium quaternaire.

**18.** Composition selon l'une quelconque des revendications 15 à 17, caractérisée par le fait que les vésicules contiennent en plus du cholestérol.

**19.** Composition selon l'une quelconque des revendications 1 à 18, caractérisée en ce que le composé filtrant les radiations UV et comportant au moins une fonction acide, libre ou au moins partiellement neutralisée, est un filtre hydrosoluble ayant au moins un radical sulfonique $-SO_3H$

**20.** Composition selon l'une quelconque des revendications 1 à 19, caractérisée en ce que le composé filtrant le rayonnement ultraviolet est un composé de structure 3-benzylidène 2-bornanone.

**21.** Composition selon l'une quelconque des revendications 1 à 19, caractérisée en ce que le composé filtrant le rayonnement ultraviolet répond à la formule (I) suivante :

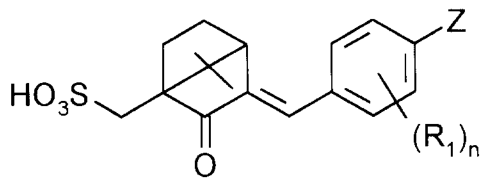

(I)

dans laquelle :

- Z désigne un groupement de formule :

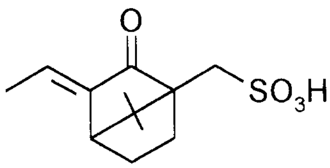

- n désigne 0 ou un nombre entier supérieur ou égal à 1 et inférieur ou égal à 4
- $R_1$ représente un ou plusieurs radicaux alkyle ou alkoxy, identiques ou différents, linéaires ou ramifiés, contenant de 1 à 4 atomes de carbone.

**22.** Composition selon la revendication 21, caractérisée en ce que le composé de formule (I) est l'acide benzène 1,4 [di(3-méthylidènecampho-10-sulfonique)].

**23.** Composition selon l'une quelconque des revendications 1 à 19, caractérisé en ce que le composé filtrant le rayonnement ultraviolet répond à la formule (II) suivante :

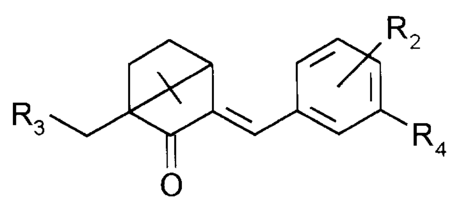

(II)

dans laquelle

- $R_2$ désigne un atome d'hydrogène, un atome d'halogéne, un radical alkyle contenant 1 à 4 atomes de carbone, un radical $-SO_3H$.
- $R_3$ et $R_4$ désignent un atome d'hydrogène ou un radical $-SO_3H$, au moins un des radicaux $R_2$, $R_3$ ou $R_4$ désignant le radical $-SO_3H$, $R_2$ et $R_4$ ne pouvant désigner simultanément un radical-$SO_3H$.

**24.** Composition selon la revendication 23, caractérisée en ce que dans la formule (II) $R_2$ désigne le radical $-SO_3H$ en position para du benzylidène camphre et $R_3$ et $R_4$ représentent chacun un atome d'hydrogène.

**25.** Composition selon la revendication 23, caractérisée en ce que dans la formule (II) $R_2$ et $R_4$ représente chacun un atome d'hydrogène et $R_3$ désigne $-SO_3H$.

**26.** Composition selon la revendication 23, caractérisée en ce que dans la formule (II) $R_2$ désigne le radical méthyle en position para du benzylidène camphre, $R_4$ un radical représente le radical $SO_3H$ et $R_3$ un atome d'hydrogène.

**27.** Composition selon la revendication 23, caractérisée en ce que dans la formule (II) $R_2$ désigne un atome de chlore en position para du benzylidène camphre, $R_4$ le radical - $SO_3H$ et $R_3$ un atome d'hydrogène.

**28.** Composition selon la revendication 23, caractérisée en ce que dans la formule (II) $R_2$ désigne le radical méthyle en position para du benzylidène camphre, $R_4$ désigne un atome d'hydrogène et $R_3$ désigne le radical $-SO_3H$.

**29.** Composition selon l'une quelconque des revendications 1 à 19, caractérisé en ce que le composé filtrant le rayonnement ultraviolet répond à la formule (III) suivante :

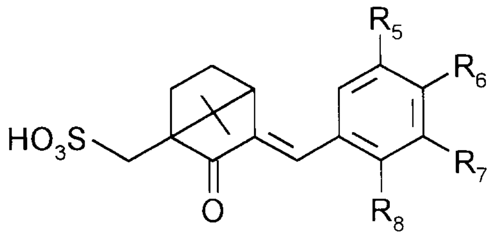

(III)

dans laquelle :

- $R_5$ et $R_7$, identiques ou différents, désignent un atome d'hydrogène, un radical hydroxyle, un radical alkyle linéaire ou ramifié contenant 1 à 8 atomes de carbone ou un radical alcoxy linéaire ou ramifié contenant 1 à 8 atomes de carbone, l'un au moins des radicaux $R_5$ et $R_7$ représentant un radical hydroxyle, alkyle ou alcoxy,
- $R_6$ et $R_8$, identiques ou différents, désignent un atome d'hydrogène, un radical hydroxyle, l'un au moins des radicaux $R_6$ et $R_8$ désignant le radical hydroxyle,
- sous réserve que lorsque $R_5$ et $R_8$ désignent l'hydrogène et que $R_6$ désigne le radical hydroxyle, $R_7$ ne désigne pas un radical alcoxy ou un atome d'hydrogène.

**30.** Composition selon la revendication 29, caractérisée en ce que dans la formule (III) $R_5$ est un radical méthyle, $R_6$ un atome d'hydrogène, $R_7$ un radical tertiobutyle et $R_8$ un radical hydroxyle.

**31.** Composition selon la revendication 29, caractérisée en ce que dans la formule (III) $R_5$ est un radical méthoxy, $R_6$ un atome d'hydrogène, $R_7$ un radical tertiobutyle et $R_8$ un radical hydroxyle.

**32.** Composition selon la revendication 29, caractérisée en ce que dans la formule (III) $R_5$ et $R_7$ désignent chacun un radical tertiobutyle, $R_6$ un radical hydroxyle et $R_8$ un atome d'hydrogène.

**33.** Composition selon l'une quelconque des revendications 1 à 19, caractérisé en ce que le composé filtrant le rayonnement ultraviolet répond à la formule (IV) suivante :

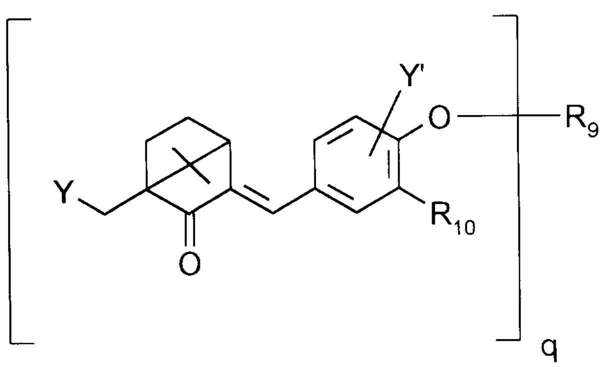

(IV)

dans laquelle

- $R_9$ désigne un atome d'hydrogène, un radical alkyle linéaire ou ramifié contenant de 1 à 18 atomes de carbone , un radical alcényle, linéaire ou ramifié, contenant de 3 à 18 atomes de carbone, un groupement choisi parmi le groupe comprenant :

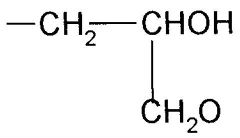

$-(CH_2CH_2O)_n-H$, ou $-CH_2-CHOH-CH_3$,

ou un radical divalent : $-(CH_2)_m-$ ou $-CH_2$ -CHOH-$CH_2$-
n étant un nombre entier compris entre 1 et 6 ($1 \leq n \leq 6$) et m un nombre entier compris entre 1 et 10 ($1 \leq m \leq 10$).

- $R_{10}$ désigne un atome d'hydrogène, un radical alcoxy contenant de 1 à 4 atomes de carbone, ou un radical divalent -O- relié au radical $R_9$ lorsque celui-ci est divalent lui aussi.
- q désigne un nombre entier égal à 1 ou 2, étant entendu que si q est égal à 2, $R_9$ doit désigner un radical divalent.
- Y et Y' désignent un atome d'hydrogène ou un radical $-SO_3H$, au moins un de ces radicaux Y ou Y' est différent de l'hydrogène.

**34.** Composition selon la revendication 33, caractérisée en ce que dans la formule (IV) q est égal à 1, Y et $R_{10}$ désignent chacun un atome d'hydrogène, $R_9$ désigne un radical méthyle, Y' en position 3 désigne un radical $-SO_3H$.

**35.** Composition selon la revendication 33, caractérisée en ce que dans la formule (IV) q est égal à 1, Y désigne un radical $-SO_3H$, Y' un atome d'hydrogène; $R_{10}$ un radical divalent -O- relié à $R_9$ désignant un radical méthylène.

**36.** Composition selon la revendication 33, caractérisée en ce que dans la formule (IV) q est égal à 1, Y désigne un radical $-SO_3H$, Y' et $R_{10}$ désignent tous deux un atome d'hydrogène; $R_9$ désigne un radical méthyle.

**37.** Composition selon la revendication 33, caractérisée en ce que dans la formule (IV) q est égal à 1, Y désigne un radical $-SO_3H$, Y' un atome d'hydrogène; $R^9$ désigne un radical méthyle, $R_{10}$ désigne un radical méthoxy.

**38.** Composition selon la revendication 33, caractérisée en ce que dans la formule (IV) q est égal à 1, Y désigne un radical $-SO_3H$, Y' et $R_{10}$ désignent tous deux un atome d'hydrogène; $R_9$ un radical n butyle.

**39.** Composition selon la revendication 33, caractérisée en ce que dans la formule (IV) q est égal à 1, Y désigne un radical $-SO_3H$, Y' un atome d'hydrogène; $R_9$ désigne un radical n butyle, $R_{10}$ un radical méthoxy

**40.** Composition selon l'une quelconque des revendications 1 à 19, caractérisé en ce que le composé filtrant le rayonnement ultraviolet répond à la formule (V) suivante :

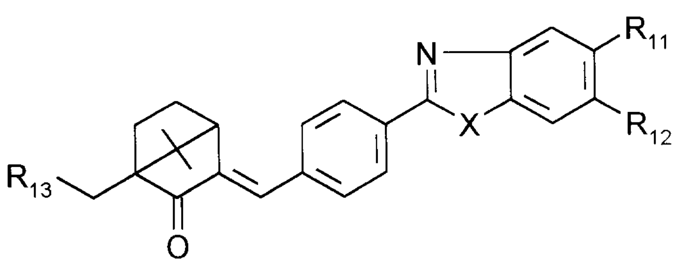

(V)

dans laquelle :

- $R_{11}$ désigne un atome d'hydrogène, un radical alkyle ou alcoxy, linéaire ou ramifié, contenant 1 à 6 atomes de carbone, un radical $-SO_3H$.
- $R_{12}$ désigne un atome d'hydrogène, un radical alkyle ou alcoxy linéaire ou ramifié contenant 1 à 6 atomes de carbone.
- $R_{13}$ désigne un atome d'hydrogène, ou un radical $-SO_3H$.
- l'un au moins des radicaux $R_{11}$ ou $R_{13}$ désigne un radical $-SO_3H$.

- X est un atome d'oxygène ou de soufre, ou un groupement -NR-, R étant un atome d'hydrogène ou un radical alkyle linéaire ou ramifié contenant de 1 à 6 atomes de carbone.

**41.** Composition selon la revendication 40, caractérisée en ce que dans la formule (V) X désigne un radical -NH-, $R_{11}$désigne un radical -$SO_3H$, $R_{12}$ et $R_{13}$ désignent tous deux un atome d'hydrogène.

**42.** Composition selon l'une quelconque des revendications 1 à 19, caractérisé en ce que le composé filtrant le rayonnement ultraviolet répond à la formule (VI) suivante :

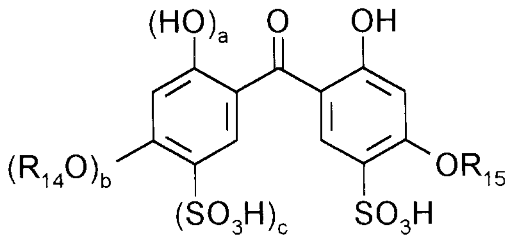

(VI)

dans laquelle

- $R_{14}$ et $R_{15}$ identiques ou différents, désignent un atome d'hydrogène ou un radical alkyle, linéaire ou ramifié, contenant de 1 à 8 atomes de carbone;
- a, b et c identiques ou différents sont égaux à 0 ou 1.

**43.** Composition selon la revendication 42, caractérisée en ce que dans la formule (VI) a=b=c=0 et $R_{15}$ désigne un radical méthyle

**44.** Composition selon l'une quelconque des revendications 1 à 19, caractérisé en ce que le composé filtrant le rayonnement ultraviolet répond à la formule (VII) suivante :

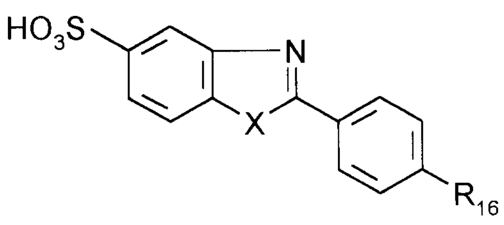

(VII)

dans laquelle

- X désigne un atome d'oxygène ou un radical -NH-
- $R_{16}$ désigne un atome d'hydrogène, un radical alkyle ou alcoxy, linéaire ou ramifié, contenant de 1 à 8 atomes de carbone ou un groupement de formule (VIII)

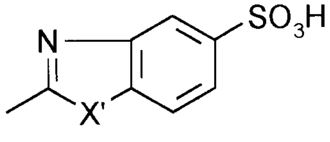

(VIII)

dans laquelle X' désigne indépendamment de X, un atome oxygène ou un radical -NH-

**45.** Composition selon la revendication 44, caractérisée en ce que dans la formule (VII) X désigne le radical -NH-, et $R_{16}$ désigne un atome d'hydrogène.

**46.** Composition selon la revendication 44, caractérisée en ce que dans la formule (VII) X désigne le radical -NH-, $R_{16}$ désigne le groupement de formule (VIII) avec X' désignant le radical -NH-.

**47.** Composition selon la revendication 44, caractérisée en ce que dans la formule (VII) X désigne un atome d'oxygène, $R_{16}$ désigne le groupement de formule (VIII) avec X' désignant un atome d'oxygène.

**48.** Composition selon l'une quelconque des revendications 1 à 47, caractérisée par le fait que les composés filtres à fonction acide, sont présents dans des concentrations allant de 0,1 à 10% en poids en matière active par rapport au poids total de la composition et plus particulièrement de 0,5 à 5 % en poids en matière active.

**49.** Composition selon l'une quelconque des revendications 1 à 48, caractérisée par le fait qu'elle contient une huile dispersée dans celle-ci par les vésicules lipidiques et éventuellement au moins un actif cosmétique ou dermatologique liposoluble présent dans la phase huileuse.

**50.** Composition selon l'une quelconque des revendications 1 à 49, caractérisée par le fait qu'elle contient en plus dans la phase aqueuse continue au moins un actif cosmétique ou dermatologique hydrosoluble.

**51.** Composition selon l'une quelconque des revendications 1 à 50, caractérisée par le fait qu'elle contient en plus des adjuvants pris dans le groupe formé par les gélifiants, les conservateurs, les colorants, les opacifiants et les parfums.

**52.** Composition à usage topique, caractérisée par le fait qu'elle est constituée par une composition selon l'une quelconque des revendications 1 à 51.

**53.** Utilisation non thérapeutique d'une composition selon l'une quelconque des revendications 1 à 51 comme base de produit pour le soin de la peau et/ou du cuir chevelu et/ou des cheveux et/ou des cils ou des sourcils.

**54.** Utilisation d'une composition selon l'une quelconque des revendications 1 à 51 comme base de produit pour le maquillage.

**55.** Utilisation non thérapeutique d'une composition selon l'une quelconque des revendications 1 à 51 comme base de produit pour la protection de l'épiderme humain, des cheveux, du cuir chevelu, des cils ou des sourcils contre les rayons ultraviolets.

**56.** Procédé de traitement non-thérapeutique de la peau, du cuir chevelu des cheveux, des cils ou des sourcils destiné à les protéger contre les effets des rayons UV consistant à appliquer sur ceux-ci une quantité efficace d'une composition cosmétique selon l'une quelconque des revendications 1 à 51.

## Claims

**1.** Composition comprising an aqueous dispersion of lipid vesicles encapsulating a compound which screens out UV radiation and contains at least one free or at least partially neutralized acidic function, characterized in that the lipid vesicles have a lipid membrane formed from at least one nonionic amphiphilic lipid (A) or from at least one hydrocarbon ionic amphiphilic lipid (B) having an iodine number of less than 10, and from at least one totally neutralized ionic amphiphilic lipid (C).

**2.** Composition according to Claim 1, characterized in that it is an oil-in-water dispersion in which the lipid vesicles act as an agent for dispersing the oil in the continuous aqueous phase of the dispersion.

**3.** Composition according to Claim 1 or 2, characterized in that the vesicles are lipid vesicles with an aqueous core (encapsulating an aqueous phase).

**4.** Composition according to any one of Claims 1 to 3, characterized in that the nonionic amphiphilic lipids (A) forming the membrane of the vesicles are chosen from the group formed by:

(i) esters and/or ethers of polyol and of fatty acid, which may or may not be polyoxyethylenated;
(ii) $\alpha$-butylglycoside esters and/or ethers of fatty acid.

**5.** Composition according to Claim 4, characterized in that the nonionic amphiphilic lipids (A) are chosen from mixtures of esters and/or mixtures of ethers of at least one polyol chosen from the group formed by polyethylene glycol containing from 1 to 60 units of ethylene oxide, sorbitan, sorbitan containing 2 to 60 units of ethylene oxide, glycerol containing 2 to 30 units of ethylene oxide, polyglycerols containing 2 to 15 units of glycerol, sucroses, glucoses containing 2 to 30 units of ethylene oxide, and at least one fatty acid containing a saturated or unsaturated, linear or branched $C_5$-$C_{22}$ alkyl chain, the number of alkyl chains per polyol group being between 1 and 10.

**6.** Composition according to Claim 4, characterized in that the nonionic amphiphilic lipids (A) are chosen from α-butylglucoside esters and/or ethers of fatty acid containing a fatty chain having from 8 to 24 carbon atoms, more preferably from 12 to 22 carbon atoms and more particularly from 14 to 18 carbon atoms.

**7.** Composition according to Claim 6, characterized in that the nonionic amphiphilic lipids (A) are chosen from the group consisting of α-butylglucoside esters and/or ethers of lauric acid ($C_{12}$), of myristic acid ($C_{14}$), of palmitic acid ($C_{16}$), of stearic acid ($C_{18}$) and of behenic acid ($C_{22}$).

**8.** Composition according to Claim 6 or 7, characterized in that the nonionic amphiphilic lipids (A) are a mixture of α-butylglucoside mono- and diester of palmitic acid.

**9.** Composition according to any one of Claims 6 to 8, characterized in that the nonionic amphiphilic lipids (A) forming the membrane of the vesicles are chosen from the group formed by mixtures of α-butylglucoside esters and/or mixtures of α-butylglucoside ethers of different fatty acids in which the various fatty chains contain, relative to each other, a similar number of carbon atoms and mixtures of α-butylglucoside mono-, di-, tri- or polyesters and/or α-butylglucoside mono-, di-, tri- or polyethers of the same fatty acid.

**10.** Composition according to any one of Claims 1 to 9, characterized in that the saturated hydrocarbon ionic amphiphilic lipids (B) forming the membrane of the vesicles are chosen from the group formed by synthetic phospholipids or phospholipids modified chemically or enzymatically.

**11.** Composition according to any one of Claims 1 to 10, characterized in that the totally neutralized ionic amphiphilic lipids (C) forming the membrane of the vesicles are chosen from those having totally neutralized acidic functions.

**12.** Composition according to Claim 11, characterized in that the totally neutralized ionic amphiphilic lipids (C) forming the membrane of the vesicles are chosen from the group consisting of:

(i) disodium or dipotassium acylglutamates;
(ii) alkali metal salts of dicetyl phosphate and of dimyristyl phosphate;
(iii) phospholipids;
(iv) alkylsulphonic derivatives of formula:

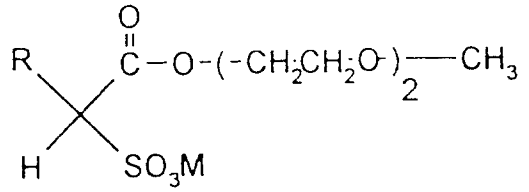

in which formula R represents $C_{12}$-$C_{22}$ radicals, in particular $C_{16}H_{33}$ and $C_{18}H_{37}$ radicals, taken as a mixture or separately, and M is an alkali metal;
(v) salicylic acid derivatives of formula (1):

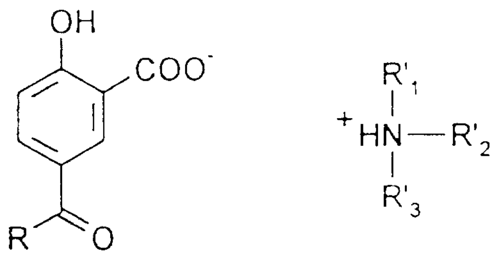

(1)

in which R represents a linear or branched $C_{11}$-$C_{17}$ alkyl radical; $R'_1$, $R'_2$ and $R'_3$, which may be identical or different, represent a $C_1$-$C_{18}$ alkyl or hydroxyalkyl radical, it being possible for one of the radicals $R'_1$, $R'_2$ and $R'_3$ to be a benzyl radical;

(vi) alkali metal salts of cholesteryl sulphate;

(vii) alkali metal salts of cholesteryl phosphate;

(viii) alkali metal salts of phosphatidic acid.

**13.** Composition according to Claim 12, characterized in that the totally neutralized ionic amphiphilic lipids (C) forming the membrane of the vesicles are chosen from the group consisting of the disodium salt of N-stearoylglutamic acid and N,N-dimethyl-N-(2-hydroxyethyl)ammonium 5-n-dodecanoylsalicylate.

**14.** Composition according to any one of Claims 1 to 13, characterized in that the weight ratio between the amount of nonionic amphiphilic lipid (A) or of saturated hydrocarbon ionic amphiphilic lipid (B) and the amount of totally neutralized ionic amphiphilic lipid (C) is between 50/1 and 50/25 and the weight ratio between the amount of lipid phase and the amount of aqueous phase in the dispersion is between 1/1000 and 300/1000.

**15.** Composition according to any one of Claims 1 to 14, characterized in that the vesicles also contain one or more water-soluble, liposoluble or amphiphilic active compound(s) having cosmetic and/or dermopharmaceutical activity.

**16.** Composition according to any one of Claims 1 to 15, characterized in that the vesicles also contain at least one additive whose main function is to reduce the permeability of the vesicles, to prevent their flocculation and their fusion and to increase the degree of encapsulation.

**17.** Composition according to Claim 16, characterized in that the vesicles also contain at least one additive chosen from the group formed by:

- sterols,
- long-chain alcohols and diols,
- long-chain amines and their quaternary ammonium derivatives.

**18.** Composition according to any one of Claims 15 to 17, characterized in that the vesicles also contain cholesterol.

**19.** Composition according to any one of Claims 1 to 18, characterized in that the compound which screens out UV radiation and which contains at least one free or at least partially neutralized acidic function is a water-soluble screening agent which has at least one sulphonic radical $-SO_3H$.

**20.** Composition according to any one of Claims 1 to 19, characterized in that the compound which screens out ultraviolet radiation is a compound of 3-benzylidene-2-bornanone structure.

**21.** Composition according to any one of Claims 1 to 19, characterized in that the compound which screens out ultraviolet radiation corresponds to formula (I) below:

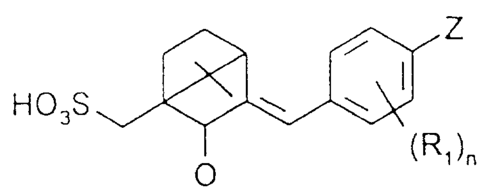

(I)

in which:

- Z denotes a group of formula

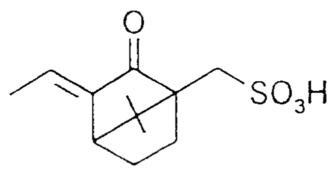

- n denotes 0 or is an integer greater than or equal to 1 and less than or equal to 4
- $R_1$ represents one or more linear or branched alkyl or alkoxy radicals, which may be identical or different, containing from 1 to 4 carbon atoms.

**22.** Composition according to Claim 21, characterized in that the compound of formula (I) is benzene-1,4-[di(3-methylidene-10-camphorsulphonic)] acid.

**23.** Composition according to any one of Claims 1 to 19, characterized in that the compound which screens out ultraviolet radiation corresponds to formula (II) below:

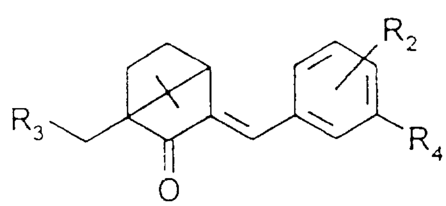

(II)

in which

- $R_2$ denotes a hydrogen atom, a halogen atom, an alkyl radical containing from 1 to 4 carbon atoms or an -$SO_3H$ radical.
- $R_3$ and $R_4$ denote a hydrogen atom or an -$SO_3H$ radical, at least one of the radicals $R_2$, $R_3$ or $R_4$ denoting an -$SO_3H$ radical, $R_2$ and $R_4$ not being able simultaneously to denote an -$SO_3H$ radical.

**24.** Composition according to Claim 23, characterized in that, in formula (II), $R_2$ denotes an -$SO_3H$ radical in the para position of benzylidenecamphor and $R_3$ and $R_4$ each represent a hydrogen atom.

**25.** Composition according to Claim 23, characterized in that, in formula (II), $R_2$ and $R_4$ each represent a hydrogen atom and $R_3$ denotes -$SO_3H$.

**26.** Composition according to Claim 23, characterized in that, in formula (II), $R_2$ denotes a methyl radical in the para position of benzylidenecamphor, $R_4$ represents an $SO_3H$ radical and $R_3$ represents a hydrogen atom.

**27.** Composition according to Claim 23, characterized in that, in formula (II), $R_2$ denotes a chlorine atom in the para position of benzylidenecamphor, $R_4$ denotes an -$SO_3H$ radical and $R_3$ denotes a hydrogen atom.

**28.** Composition according to Claim 23, characterized in that, in formula (II), $R_2$ denotes a methyl radical in the para position of benzylidenecamphor, $R_4$ denotes a hydrogen atom and $R_3$ denotes an $-SO_3H$ radical.

**29.** Composition according to any one of Claims 1 to 19, characterized in that the compound which screens out ultraviolet radiation corresponds to formula (III) below:

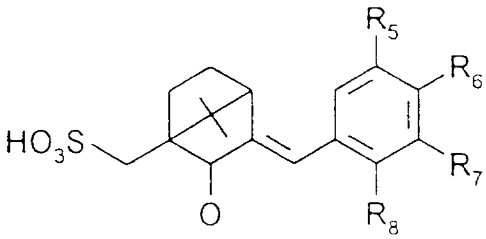

(III)

in which:

- $R_5$ and $R_7$, which may be identical or different, denote a hydrogen atom, a hydroxyl radical, a linear or branched alkyl radical containing from 1 to 8 carbon atoms or a linear or branched alkoxy radical containing from 1 to 8 carbon atoms, at least one of the radicals $R_5$ and $R_7$ representing a hydroxyl, alkyl or alkoxy radical,
- $R_6$ and $R_8$, which may be identical or different, denote a hydrogen atom or a hydroxyl radical, at least one of the radicals $R_6$ and $R_8$ denoting a hydroxyl radical,
- with the proviso that when $R_5$ and $R_8$ denote a hydrogen atom and when $R_6$ denotes a hydroxyl radical, $R_7$ cannot denote an alkoxy radical or a hydrogen atom.

**30.** Composition according to Claim 29, characterized in that, in formula (III), $R_5$ is a methyl radical, $R_6$ is a hydrogen atom, $R_7$ is a tert-butyl radical and $R_8$ is a hydroxyl radical.

**31.** Composition according to Claim 29, characterized in that, in formula (III), $R_5$ is a methoxy radical, $R_6$ is a hydrogen atom, $R_7$ is a tert-butyl radical and $R_8$ is a hydroxyl radical.

**32.** Composition according to Claim 29, characterized in that, in formula (III), $R_5$ and $R_7$ each denote a tert-butyl radical, $R_6$ denotes a hydroxyl radical and $R_8$ denotes a hydrogen atom.

**33.** Composition according to any one of Claims 1 to 19, characterized in that the compound which screens out ultraviolet radiation corresponds to formula (IV) below:

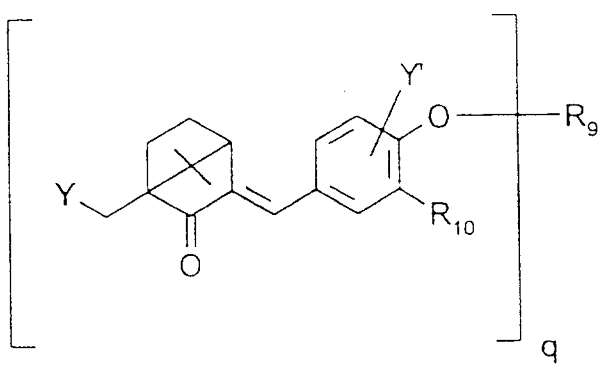

(IV)

in which:

- $R_9$ denotes a hydrogen atom, a linear or branched alkyl radical containing from 1 to 18 carbon atoms, a linear or branched alkenyl radical containing from 3 to 18 carbon atoms, a group chosen from the group comprising:

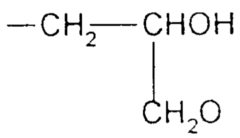 $-(CH_2CH_2O)_n-H$, ou $-CH_2-CHOH-CH_3$,

or a divalent radical: $-(CH_2)_m-$ or $-CH_2-CHOH-CH_2-$
n being an integer between 1 and 6 ($1 \leq n \leq 6$) and m an integer between 1 and 10 ($1 \leq m \leq 10$),

- $R_{10}$ denotes a hydrogen atom, an alkoxy radical containing from 1 to 4 carbon atoms or a divalent radical -O- connected to the radical $R_9$ when the latter is also divalent,
- q denotes an integer equal to 1 or 2, it being understood that if q is equal to 2, $R_9$ must denote a divalent radical,
- Y and Y' denote a hydrogen atom or an $-SO_3H$ radical, at least one of these radicals Y or Y' being other than hydrogen.

**34.** Composition according to Claim 33, characterized in that, in formula (IV), q is equal to 1, Y and $R_{10}$ each denote a hydrogen atom, $R_9$ denotes a methyl radical, and Y' in position 3 denotes an $-SO_3H$ radical.

**35.** Composition according to Claim 33, characterized in that, in formula (IV), q is equal to 1, Y denotes an $-SO_3H$ radical, Y' denotes a hydrogen atom and $R_{10}$ denotes a divalent radical -O- connected to $R_9$ denoting a methylene radical.

**36.** Composition according to Claim 33, characterized in that, in formula (IV), q is equal to 1, Y denotes an $-SO_3H$ radical, Y' and $R_{10}$ both denote a hydrogen atom and $R_9$ denotes a methyl radical.

**37.** Composition according to Claim 33, characterized in that, in formula (IV), q is equal to 1, Y denotes an $-SO_3H$ radical, Y' denotes a hydrogen atom, $R_9$ denotes a methyl radical and $R_{10}$ denotes a methoxy radical.

**38.** Composition according to Claim 33, characterized in that, in formula (IV), q is equal to 1, Y denotes an $-SO_3H$ radical, Y' and $R_{10}$ both denote a hydrogen atom and $R_9$ denotes an n-butyl radical.

**39.** Composition according to Claim 33, characterized in that, in formula (IV), q is equal to 1, Y denotes an $-SO_3H$ radical, Y' denotes a hydrogen atom, $R_9$ denotes an n-butyl radical and $R_{10}$ denotes a methoxy radical.

**40.** Composition according to any one of Claims 1 to 19, characterized in that the compound which screens out ultraviolet radiation corresponds to formula (V) below:

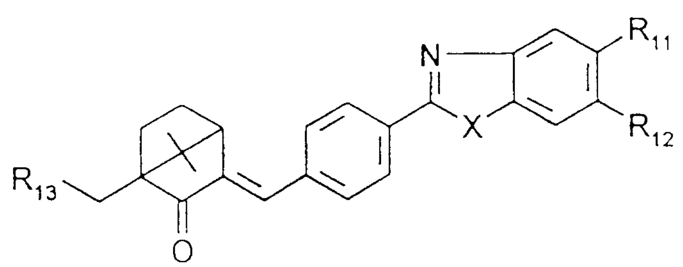 (V)

in which:

- $R_{11}$ denotes a hydrogen atom, a linear or branched alkyl or alkoxy radical containing from 1 to 6 carbon atoms or an $-SO_3H$ radical,
- $R_{12}$ denotes a hydrogen atom or a linear or branched alkyl or alkoxy radical containing from 1 to 6 carbon atoms,
- $R_{13}$ denotes a hydrogen atom or an $-SO_3H$ radical,
- at least one of the radicals $R_{11}$ and $R_{13}$ denotes an $-SO_3H$ radical,

- X is an oxygen or sulphur atom or a group -NR-, R being a hydrogen atom or a linear or branched alkyl radical containing from 1 to 6 carbon atoms.

**41.** Composition according to Claim 40, characterized in that, in formula (V), X denotes an -NH- radical, $R_{11}$ denotes an $-SO_3H$ radical and $R_{12}$ and $R_{13}$ both denote a hydrogen atom.

**42.** Composition according to any one of Claims 1 to 19, characterized in that the compound which screens out ultraviolet radiation corresponds to formula (VI) below:

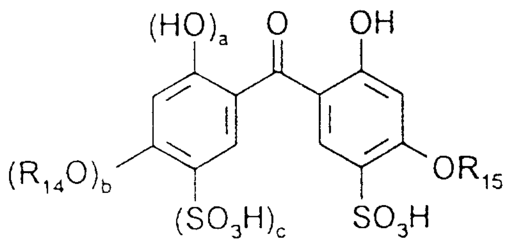

(VI)

in which:

- $R_{14}$ and $R_{15}$, which may be identical or different, denote either a hydrogen atom or a linear or branched alkyl radical containing from 1 to 8 carbon atoms.
- a, b and c, which may be identical or different, are equal to 0 or 1.

**43.** Composition according to Claim 42, characterized in that, in formula (VI), a=b=c=0 and $R_{15}$ denotes a methyl radical.

**44.** Composition according to any one of Claims 1 to 19, characterized in that the compound which screens out ultraviolet radiation corresponds to formula (VII) below:

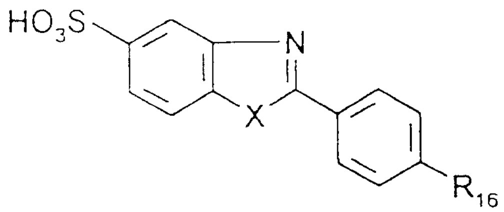

(VII)

in which:

- X denotes an oxygen atom or an -NH- radical
- $R_{16}$ denotes a hydrogen atom, a linear or branched alkyl or alkoxy radical containing from 1 to 8 carbon atoms or a group of formula

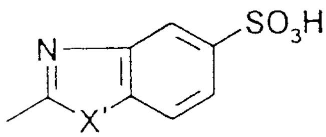

(VIII)

in which X' denotes, independently of X, an oxygen atom or an -NH- radical.

**45.** Composition according to Claim 44, characterized in that, in formula (VII), X denotes an -NH- radical and $R_{16}$ denotes a hydrogen atom.

**46.** Composition according to Claim 44, characterized in that, in formula (VII), X denotes an -NH- radical and $R_{16}$ denotes a group of formula (VIII) with X' denoting an -NH- radical.

**47.** Composition according to Claim 44, characterized in that, in formula (VII), X denotes an oxygen atom and $R_{16}$ denotes a group of formula (VIII) with X' denoting an oxygen atom.

**48.** Composition according to any one of Claims 1 to 47, characterized in that the screening compounds with acidic functionality are present in concentrations ranging from 0.1 to 10 % by weight of active substance relative to the total weight of the composition and more particularly from 0.5 to 5 % by weight of active substance.

**49.** Composition according to any one of Claims 1 to 48, characterized in that it contains an oil dispersed in the latter by lipid vesicles and optionally at least one liposoluble cosmetic or dermatological active agent present in the oily phase.

**50.** Composition according to any one of Claims 1 to 49, characterized in that it also contains, in the continuous aqueous phase, at least one water-soluble cosmetic or dermatological active agent.

**51.** Composition according to any one of Claims 1 to 50, characterized in that it also contains adjuvants taken from the group formed by gelling agents, preserving agents, dyes, opacifiers and fragrances.

**52.** Composition for topical use, characterized in that it consists of a composition according to any one of Claims 1 to 51.

**53.** Non-therapeutic use of a composition according to any one of Claims 1 to 51 as a base of a product to care for the skin and/or the scalp and/or the hair and/or the eyelashes or the eyebrows.

**54.** Use of a composition according to any one of Claims 1 to 51 as a base for a make-up product.

**55.** Non-therapeutical use of a composition according to any one of Claims 1 to 51 as a base for a product to protect the human epidermis, the hair, the scalp, the eyelashes or the eyebrows against ultraviolet rays.

**56.** Process for the non-therapeutic treatment of the skin, the scalp, the hair, the eyelashes or the eyebrows which is intended to protect them against the effects of UV rays, this process consisting in applying to the latter an effective amount of a cosmetic composition according to any one of Claims 1 to 51.

**Patentansprüche**

**1.** Zusammensetzungen, die eine wässerige Dispersion von Lipidvesikeln enthalten, welche eine UV-Strahlung filternde Verbindung einkapseln, die mindestens eine freie oder zumindest teilweise neutralisierte Säurefunktion aufweist, dadurch gekennzeichnet, daß die Lipidvesikel eine Lipidmembran aufweisen, die aus mindestens einem nichtionischen amphiphilen Lipid (A) oder aus mindestens einem ionischen amphiphilen Kohlenwasserstoff-Lipid (B), dessen lodzahl kleiner 10 ist, und aus mindestens einem vollständig neutralisierten ionischen amphiphilen Lipid (C) besteht.

**2.** Zusammensetzungen nach Anspruch 1, dadurch gekennzeichnet, daß sie Öl-in-Wasser-Dispersionen sind, in denen die Lipidvesikel als Dispergiermittel fuhr das Öl in der kontinuierlichen wässerigen Phase der Dispersion fungieren.

**3.** Zusammensetzungen nach Anspruch 1 oder 2, dadurch gekennzeichnet, daß die Vesikel Lipidvesikel mit wässerigem inneren Kompartiment sind (Lipidvesikel, die eine wässerige Phase einkapseln).

**4.** Zusammensetzungen nach einem der Ansprüche 1 bis 3, dadurch gekennzeichnet, daß die nichtionischen amphiphilen Lipide (A), die die Membran der Vesikel bilden, unter folgenden Lipiden ausgewählt sind:

(i) den gegebenenfalls polyethoxylierten Estern und/oder den gegebenenfalls polyethoxylierten Ethern aus einem Polyol und einer Fettsäure
und
(ii) den Estern und/oder den Ethern aus einer Fettsäure und einem $\alpha$-Butylglykosid.

5. Zusammensetzungen nach Anspruch 4, dadurch gekennzeichnet, daß die nichtionischen amphiphilen Lipide (A) unter den Gemischen von Estern und/oder den Gemischen von Ethern ausgewählt sind, die aus mindestens einem Polyol, das unter dem Polyethylenglykol mit 1 bis 60 Ethylenoxideinheiten, dem Sorbitan und dem Sorbitan, das 2 bis 60 Ethylenoxideinheiten trägt, dem Glycerin, das 2 bis 30 Ethylenoxideinheiten trägt, den Polyglycerinen mit 2 bis 15 Glycerineinheiten, den Saccharosen und den Glucosen, die 2 bis 30 Ethylenoxideinheiten tragen, ausgewählt sind, und mindestens einer Fettsäure, die eine gesättigte oder ungesättigte und geradkettige oder verzweigte $C_{5-22}$-Alkylkette aufweist, gebildet werden, wobei die Anzahl der Alkylketten pro Polyolgruppe im Bereich von 1 bis 10 liegt.

6. Zusammensetzungen nach Anspruch 4, dadurch gekennzeichnet, daß die nichtionischen amphiphilen Lipide (A) unter den Estern und/oder den Ethern aus einer Fettsäure und einem α-Butylglucosid ausgewählt sind, die eine Fettsäurekette mit 8 bis 24 Kohlenstoffatomen, vorzugsweise mit 12 bis 22 Kohlenstoffatomen und insbesondere mit 14 bis 18 Kohlenstoffatomen aufweisen.

7. Zusammensetzungen nach Anspruch 6, dadurch gekennzeichnet, daß die nichtionischen amphiphilen Lipide (A) unter den Estern und/oder den Ethern aus Laurinsäure ($C_{12}$), Myristinsäure ($C_{14}$), Palmitinsäure ($C_{16}$), Stearinsäure ($C_{18}$) oder Behensäure ($C_{22}$) und α-Butylglucosid ausgewählt sind.

8. Zusammensetzungen nach Anspruch 6 oder 7, dadurch gekennzeichnet, daß die nichtionischen amphiphilen Lipide (A) ein Gemisch des Mono- und des Diesters aus Palmitinsäure und α-Butylglucosid sind.

9. Zusammensetzungen nach einem der Ansprüche 6 bis 8, dadurch gekennzeichnet, daß die nichtionischen amphiphilen Lipide (A), die die Membran der Vesikel bilden, unter folgenden Lipiden ausgewählt sind:

- den Gemischen von Estern und/oder den Gemischen von Ethern aus verschiedenen Fettsäuren und α-Butylglucosid, wobei die Fettsäureketten dieser Esterbeziehungsweise Ethergemische jeweils eine ähnliche Anzahl von Kohlenstoffatomen aufweisen, und
- den Gemischen der Mono-, Di-, Tri- und Polyester und/oder der Mono-, Di-, Tri- und Polyether aus einer einzigen Fettsäure und α-Butylglucosid.

10. Zusammensetzungen nach einem der Ansprüche 1 bis 9, dadurch gekennzeichnet, daß die gesättigten ionischen amphiphilen Kohlenwasserstofflipide (B), die die Membran der Vesikel bilden, unter den synthetischen Phospholipiden und den chemisch oder enzymatisch modifizierten Phospholipiden ausgewählt sind.

11. Zusammensetzungen nach einem der Ansprüche 1 bis 10, dadurch gekennzeichnet, daß die vollständig neutralisierten ionischen amphiphilen Lipide (C), die die Membran der Vesikel bilden, unter denjenigen mit vollständig neutralisierten Säurefunktionen ausgewählt sind.

12. Zusammensetzungen nach Anspruch 11, dadurch gekennzeichnet, daß die vollständig neutralisierten ionischen amphiphilen Lipide (C), die die Membran der Vesikel bilden, unter folgenden Lipiden ausgewählt sind:

(i) den Dinatrium- oder Dikalium-acylglutamaten;
(ii) den Alkalisalzen des Dicetylphosphats und des Dimyristylphosphats;
(iii) den Phospholipiden;
(iv) den Derivaten der Alkylsulfonsäuren der folgenden Formel:

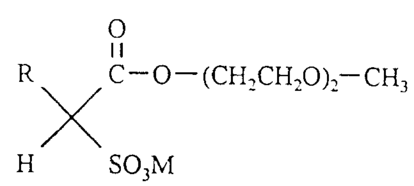

in der R einen $C_{12-22}$-Rest und insbesondere $C_{16}H_{33}$ oder $C_{18}H_{37}$ darstellt, wobei diese Reste einzeln oder gemischt vorliegen können, und in der M ein Alkalimetall ist,
(v) den Derivaten der Salicylsäure der Formel (1):

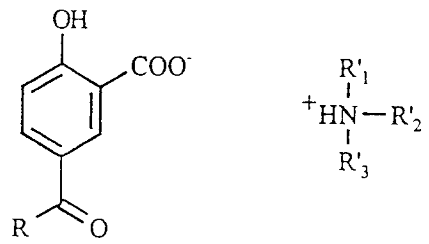

(1)

in der R einen geradkettigen oder verzweigten $C_{11-17}$-Alkylrest darstellt und und in der $R'_1$, $R'_2$ und $R'_3$, die gleich oder verschieden sein können, jeweils einen $C_{1-18}$-Alkyl- oder Hydroxylalkylrest darstellen, wobei einer der Reste $R'_1$, $R'_2$ und $R'_3$ ein Benzylrest sein kann;
(vi) den Alkalisalzen des Cholesterinsulfats;
(vii) den Alkalisalzen des Cholesterinphosphats
und
(viii) den Alkalisalzen der Phosphatidylsäure.

**13.** Zusammensetzungen nach Anspruch 12, dadurch gekennzeichnet, daß die vollständig neutralisierten ionischen amphiphilen Lipide (C), die die Membran der Vesikel bilden, unter dem Dinatriumsalz der N-Stearoyl-glutaminsäure und dem N,N-Dimethyl-N-(2-hydroxy-ethyl)-ammonium-5-n-dodecanoylsalicylat ausgewählt sind.

**14.** Zusammensetzungen nach einem der Ansprüche 1 bis 13, dadurch gekennzeichnet, daß das Gewichtsverhältnis nichtionisches amphiphiles Lipid (A) beziehungsweise gesättigtes ionisches amphiphiles Kohlenwasserstofflipid (B)/vollständig neutralisiertes ionisches amphiphiles Lipid (C) im Bereich von 50/1 bis 50/25 liegt und das Gewichtsverhältnis Lipidphase/wässerige Phase der Dispersion im Bereich von 1/1000 bis 300/1000 liegt.

**15.** Zusammensetzungen nach einem der Ansprache 1 bis 14, dadurch gekennzeichnet, daß die Vesikel ferner einen oder mehrere wasserlösliche, fettlösliche oder amphiphile Wirkstoffe enthalten, die eine kosmetische und/oder dermopharmazeutische Aktivität aufweisen.

**16.** Zusammensetzungen nach einem der Ansprüche 1 bis 15, dadurch gekennzeichnet, daß die Vesikel zusätzlich mindestens einen Hilfsstoff enthalten, dessen Hauptfunktion darin besteht, die Permeabilität der Vesikel zu verringern, ihr Ausflocken und ihre Fusion zu verhindern und den Einkapselungsgrad zu erhöhen.

**17.** Zusammensetzungen nach Anspruch 16, dadurch gekennzeichnet, daß die Vesikel zusätzlich mindestens einen Hilfsstoff enthalten, der unter folgenden Verbindungen ausgewählt ist:

- den Sterinen;
- den langkettigen Alkoholen und Diolen
  und
- den langkettigen Aminen und deren quartären Ammoniumsalzen.

**18.** Zusammensetzungen nach einem der Ansprüche 15 bis 17, dadurch gekennzeichnet, daß die Vesikel ferner Cholesterin enthalten.

**19.** Zusammensetzungen nach einem der Ansprüche 1 bis 18, dadurch gekennzeichnet, daß die UV-Strahlung filternde Verbindung, die mindestens eine freie oder zumindest teilweise neutralisierte Säurefunktion aufweist, ein wasserlöslicher UV-Filter mit mindestens einem Sulfonsäurerest $-SO_3H$ ist.

**20.** Zusammensetzungen nach einem der Ansprüche 1 bis 19, dadurch gekennzeichnet, daß die UV-Strahlung filternde Verbindung ein 3-Benzyliden-2-bornanon ist.

**21.** Zusammensetzungen nach einem der Ansprüche 1 bis 19, dadurch gekennzeichnet, daß die UV-Strahlung filternde Verbindung der folgenden Formel (I) entspricht:

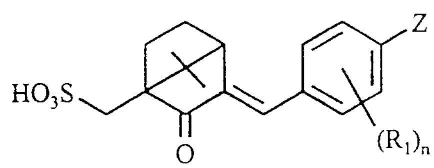

(I)

in der:

- Z eine Gruppe der folgenden Formel bezeichnet:

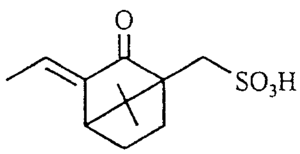

- n Null oder eine ganze Zahl größer oder gleich 1 und kleiner oder gleich 4 ist, und
- $R_1$ einen oder mehrere geradkettige oder verzweigte Alkyl- oder Alkoxyreste mit 1 bis 4 Kohlenstoffatomen darstellt, die gleich oder verschieden sein können.

**22.** Zusammensetzungen nach Anspruch 21, dadurch gekennzeichnet, daß die Verbindung der Formel (I) Benzol-1,4-[di(3-methylidencampher-10-sulfonsäure)] ist.

**23.** Zusammensetzungen nach einem der Ansprüche 1 bis 19, dadurch gekennzeichnet, daß die UV-Strahlung filternde Verbindung der folgenden Formel (II) entspricht:

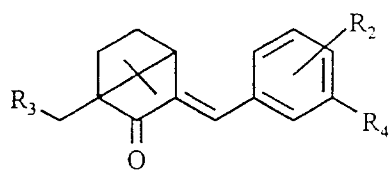

(II)

in der:

- $R_2$ ein Wasserstoffatom, ein Halogenatom, einen Alkylrest mit 1 bis 4 Kohlenstoffatomen oder einen Rest -$SO_3H$ bezeichnet und
- $R_3$ und $R_4$ jeweils ein Wasserstoffatom oder einen Rest -$SO_3H$ bezeichnen, wobei zumindest einer der Reste $R_2$, $R_3$ und $R_4$ den Rest -$SO_3H$ bezeichnet und wobei $R_2$ und $R_4$ nicht gleichzeitig den Rest -$SO_3H$ bezeichnen können.

**24.** Zusammensetzungen nach Anspruch 23, dadurch gekennzeichnet, daß in Formel (II) $R_2$ den Rest -$SO_3H$ in para-Stellung des Benzylidencamphers bezeichnet und $R_3$ und $R_4$ jeweils ein Wasserstoffatom bezeichnen.

**25.** Zusammensetzungen nach Anspruch 23, dadurch gekennzeichnet, daß in Formel (II) $R_2$ und $R_4$ jeweils ein Wasserstoffatom darstellen und $R_3$ den Rest -$SO_3H$ bezeichnet.

**26.** Zusammensetzungen nach Anspruch 23, dadurch gekennzeichnet, daß in Formel (II) $R_2$ einen Methylrest in para-Stellung des Benzylidencamphers bezeichnet, $R_4$ einen Rest -$SO_3H$ und $R_3$ ein Wasserstoffatom darstellt.

**27.** Zusammensetzungen nach Anspruch 23, dadurch gekennzeichnet, daß in Formel (II) $R_2$ ein Chloratom in para-

Stellung des Benzylidencamphers, $R_4$ den Rest $-SO_3H$ und $R_3$ ein Wasserstoffatom darstellt.

**28.** Zusammensetzungen nach Anspruch 23, dadurch gekennzeichnet, daß in Formel (II) $R_2$ den Methylrest in para-Stellung des Benzylidencamphers, $R_4$ ein Wasserstoffatom und $R_3$ den Rest $-SO_3H$ bezeichnet.

**29.** Zusammensetzungen nach einem der Ansprüche 1 bis 19, dadurch gekennzeichnet, daß die UV-Strahlung filternde Verbindung der folgenden Formel (III) entspricht:

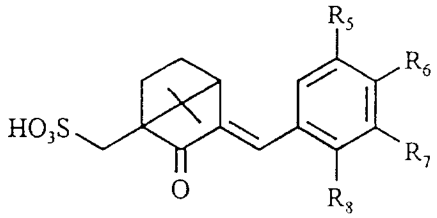

(III)

in der:

- $R_5$ und $R_7$, die gleich oder verschieden sein können, jeweils ein Wasserstoffatom, einen Hydroxylrest, einen geradkettigen oder verzweigten Alkylrest mit 1 bis 8 Kohlenstoffatomen oder einen geradketttigen oder verzweigten Alkoxyrest mit 1 bis 8 Kohlenstoffatomen darstellen, wobei zumindest einer der Reste $R_5$ und $R_7$ einen Hydroxylrest, einen Alkylrest oder einen Alkoxyrest darstellt;
- $R_6$ und $R_8$, die gleich oder verschieden sein können, jeweils ein Wasserstoffatom oder einen Hydroxylrest darstellen, wobei zumindest einer der Reste $R_6$ und $R_8$ einen Hydroxylrest bezeichnet, und
- $R_7$ unter der Voraussetzung, daß $R_5$ und $R_8$ jeweils ein Wasserstoffatom bezeichnen und $R_6$ einen Hydroxylrest bezeichnet, weder einen Alkoxyrest noch ein Wasserstoffatom bezeichnet.

**30.** Zusammensetzungen nach Anspruch 29, dadurch gekennzeichnet, daß in Formel (III) $R_5$ ein Methylrest, $R_6$ ein Wasserstoffatom, $R_7$ ein tert.-Butylrest und $R_8$ ein Hydroxylrest ist.

**31.** Zusammensetzungen nach Anspruch 29, dadurch gekennzeichnet, daß in Formel (III) $R_5$ ein Methoxyrest, $R_6$ ein Wasserstoffatom, $R_7$ ein tert.-Butylrest und $R_8$ ein Hydroxylrest ist.

**32.** Zusammensetzungen nach Anspruch 29, dadurch gekennzeichnet, daß in Formel (III) $R_5$ und $R_7$ jeweils einen tert.-Butylrest bezeichnen, $R_6$ ein Hydroxylrest ist und $R_8$ ein Wasserstoffatom ist.

**33.** Zusammensetzungen nach einem der Ansprüche 1 bis 19, dadurch gekennzeichnet, daß die UV-Strahlung filternde Verbindung der folgenden Formel (IV) entspricht:

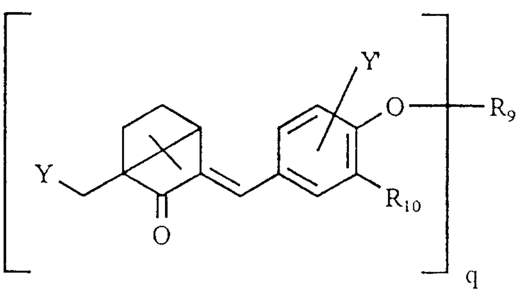

(IV)

in der:

- $R_9$ ein Wasserstoffatom, einen geradkettigen oder verzweigten Alkylrest mit 1 bis 18 Kohlenstoffatomen, einen geradkettigen oder verzweigten Alkenylrest mit 3 bis 18 Kohlenstoffatomen, eine der folgenden Gruppen:

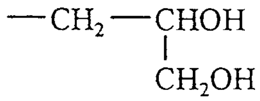 $-(CH_2CH_2O)_n-H$ oder $-CH_2-CHOH-CH_3$

oder einen zweiwertigen Rest $-(CH_2)_m-$ oder $-CH_2-CHOH-CH_2-$ bezeichnet, wobei n eine ganze Zahl von 1 bis 6 ($1 \le n \le 6$) und m eine ganze Zahl von 1 bis 10 ($1 \le m \le 10$) ist;

- $R_{10}$ ein Wasserstoffatom, einen Alkoxyrest mit 1 bis 4 Kohlenstoffatomen oder, wenn $R_9$ ein zweiwertiger Rest ist, einen an diesen Rest $R_9$ gebundenen, ebenfalls zweiwertigen Rest -O- bezeichnet;
- q eine ganze Zahl gleich 1 oder 2 bezeichnet, mit der Maßgabe, daß $R_9$ einen zweiwertigen Rest bezeichnen muß, wenn q gleich 2 ist,
  und
- Y und Y' jeweils ein Wasserstoffatom oder einen Rest $-SO_3H$ bezeichnen, wobei maximal einer dieser beiden Reste Y und Y' ein Wasserstoffatom bezeichnet.

**34.** Zusammensetzungen nach Anspruch 33, dadurch gekennzeichnet, daß in Formel (IV) q gleich 1 ist, Y und $R_{10}$ jeweils ein Wasserstoffatom bezeichnen, $R_9$ einen Methylrest und Y' einen in 3-Stellung befindlichen Rest $-SO_3H$ bezeichnet.

**35.** Zusammensetzungen nach Anspruch 33, dadurch gekennzeichnet, daß in Formel (IV) q gleich 1 ist, Y einen Rest $-SO_3H$ bezeichnet, Y' ein Wasserstoffatom bezeichnet und $R_{10}$ einen an $R_9$ gebundenen zweiwertigen Rest -O- bezeichnet, wobei $R_9$ einen Methylenrest bezeichnet.

**36.** Zusammensetzungen nach Anspruch 33, dadurch gekennzeichnet, daß in Formel (IV) q gleich 1 ist, Y einen Rest $-SO_3H$ bezeichnet, Y' und $R_{10}$ jeweils ein Wasserstoffatom bezeichnen und $R_9$ einen Methylrest bezeichnet.

**37.** Zusammensetzungen nach Anspruch 33, dadurch gekennzeichnet, daß in Formel (IV) q gleich 1 ist, Y einen Rest $-SO_3H$, Y' ein Wasserstoffatom, $R_9$ einen Methylrest und $R_{10}$ einen Methoxyrest bezeichnet.

**38.** Zusammensetzungen nach Anspruch 33, dadurch gekennzeichnet, daß in Formel (IV) q gleich 1 ist, Y einen Rest $-SO_3H$ bezeichnet, Y' und $R_{10}$ jeweils ein Wasserstoffatom bezeichnen und $R_9$ einen Butylrest bezeichnet.

**39.** Zusammensetzungen nach Anspruch 33, dadurch gekennzeichnet, daß in Formel (IV) q gleich 1 ist, Y einen Rest $-SO_3H$, Y' ein Wasserstoffatom, $R_9$ einen n-Butylrest und $R_{10}$ einen Methoxyrest bezeichnet.

**40.** Zusammensetzungen nach einem der Ansprüche 1 bis 19, dadurch gekennzeichnet, daß die UV-Strahlung filternde Verbindung der folgenden Formel (V) entspricht:

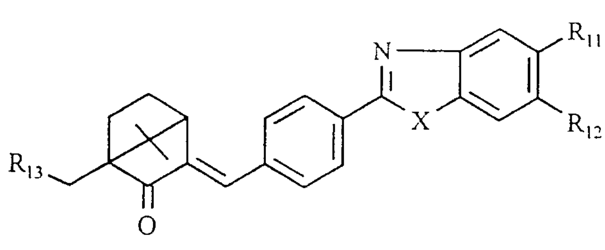

(V)

in der:

- $R_{11}$ ein Wasserstoffatom, einen geradkettigen oder verzweigten Alkyl- oder Alkoxyrest mit 1 bis 6 Kohlenstoffatomen oder einen Rest $-SO_3H$ bezeichnet;
- $R_{12}$ ein Wasserstoffatom oder einen geradkettigen oder verzweigten Alkyl- oder Alkoxyrest mit 1 bis 6 Kohlenstoffatomen bezeichnet;
- $R_{13}$ ein Wasserstoffatom oder einen Rest $-SO_3H$ bezeichnet;
- zumindest einer der Reste $R_{11}$ und $R_{13}$ einen Rest $-SO_3H$ bezeichnet; und

- X ein Sauerstoff- oder Schwefelatom oder eine Gruppe -NR- ist, wobei R in diesem Falle ein Wasserstoffatom oder ein geradkettiger oder verzweigter Alkylrest mit 1 bis 6 Kohlenstoffatomen ist.

**41.** Zusammensetzungen nach Anspruch 40, dadurch gekennzeichnet, daß in Formel (V) X einen Rest -NH- bezeichnet, $R_{11}$ einen Rest $-SO_3H$ bezeichnet und $R_{12}$ und $R_{13}$ jeweils ein Wasserstoffatom bezeichnen.

**42.** Zusammensetzungen nach einem der Ansprüche 1 bis 19, dadurch gekennzeichnet, daß die UV-Strahlung filternde Verbindung der folgenden Formel (VI) entspricht:

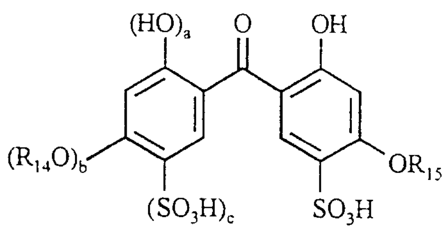

(VI)

in der:

- $R_{14}$ und $R_{15}$, die gleich oder verschieden sein können, jeweils ein Wasserstoffatom oder einen geradkettigen oder verzweigten Alkylrest mit 1 bis 8 Kohlenstoffatomen darstellen
und
- a, b und c, die gleich oder verschieden sein können, gleich 0 oder 1 sind.

**43.** Zusammensetzungen nach Anspruch 42, dadurch gekennzeichnet, daß in Formel (VI) $a = b = c = 0$ ist und $R_{15}$ einen Methylrest darstellt.

**44.** Zusammensetzungen nach einem der Ansprüche 1 bis 19, dadurch gekennzeichnet, daß die UV-Strahlung filternde Verbindung der folgenden Formel (VII) entspricht:

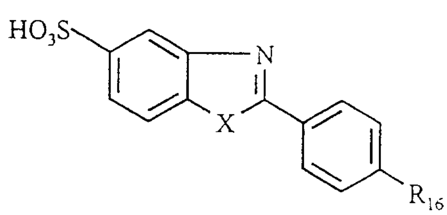

(VII)

in der :

- X ein Sauerstoffatom oder den Rest -NH- bezeichnet
und
- $R_{16}$ ein Wasserstoffatom, einen geradkettigen oder verzweigten Alkyl- oder Alkoxyrest mit 1 bis 8 Kohlenstoffatomen oder eine Gruppe der Formel (VIII) bezeichnet:

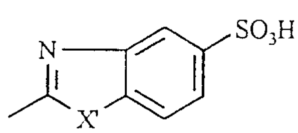

(VIII)

in der X' unabhängig von X ein Sauerstoffatom oder einen Rest -NH-bezeichnet.

**45.** Zusammensetzungen nach Anspruch 44, dadurch gekennzeichnet, daß in Formel (VII) X den Rest -NH- und $R_{16}$

ein Wasserstoffatom bezeichnet.

**46.** Zusammensetzungen nach Anspruch 44, dadurch gekennzeichnet, daß in Formel (VII) X den Rest -NH- und $R_{16}$ die Gruppe der Formel (VIII) bezeichnet, wobei X' in dieser Formel (VIII) den Rest -NH- bezeichnet.

**47.** Zusammensetzungen nach Anspruch 44, dadurch gekennzeichnet, daß in Formel (VII) X ein Sauerstoffatom und $R_{16}$ die Gruppe der Formel (VIII) bezeichnet, wobei X' in dieser Formel (VIII) ein Sauerstoffatom bezeichnet.

**48.** Zusammensetzungen nach einem der Ansprüche 1 bis 47, dadurch gekennzeichnet, daß die UV-Strahlung filternden Verbindungen mit Säurefunktion in einer Konzentration im Bereich von 0,1 bis 10 Gew.-% und insbesondere von 0,5 bis 5 Gew.-% Wirkstoff, jeweils bezogen auf das Gesamtgewicht der Zusammensetzung, vorliegen.

**49.** Zusammensetzungen nach einem der Ansprüche 1 bis 48, dadurch gekennzeichnet, daß sie ein Öl, das in dieser Zusammensetzung durch die Lipidvesikel dispergiert ist, sowie gegebenenfalls mindestens einen in der Fettphase vorliegenden fettlöslichen kosmetischen oder dermatologischen Wirkstoff enthalten.

**50.** Zusammensetzungen nach einem der Ansprüche 1 bis 49, dadurch gekennzeichnet, daß sie zusätzlich in der kontinuierlichen wässerigen Phase mindestens einen wasserlöslichen kosmetischen oder dermatologischen Wirkstoff enthalten.

**51.** Zusammensetzungen nach einem der Ansprüche 1 bis 50, dadurch gekennzeichnet, daß sie zusätzlich Hilfsstoffe enthalten, die unter den Gelbildnern, den Konservierungsmitteln, den Färbemitteln, den Tröbungsmitteln und den Parfums ausgewählt sind.

**52.** Zusammensetzungen zur topischen Anwendung, dadurch gekennzeichnet, daß sie aus einer Zusammensetzung nach einem der Ansprüche 1 bis 51 bestehen.

**53.** Nicht-therapeutische Verwendung einer Zusammensetzung nach einem der Ansprüche 1 bis 51 als Grundlage für ein Produkt zur Pflege der Haut und/oder der Kopfhaut und/oder der Haare und/oder der Wimpern oder der Augenbrauen.

**54.** Verwendung einer Zusammensetzung nach einem der Ansprüche 1 bis 51 als Grundlage für ein Schminkprodukt.

**55.** Nicht-therapeutische Verwendung einer Zusammensetzung nach einem der Ansprüche 1 bis 51 als Grundlage für ein Produkt zum Schutz der menschlichen Epidermis, der Haare, der Kopfhaut, der Wimpern oder der Augenbrauen vor Ultraviolettstrahlen.

**56.** Verfahren zur nicht-therapeutischen Behandlung der Haut, der Kopfhaut, der Haare, der Wimpern oder der Augenbrauen, das dem Schutz vor den Auswirkungen der UV-Strahlen dient, wobei dieses Verfahren darin besteht, daß auf diese eine wirksame Menge einer kosmetischen Zusammensetzung nach einem der Ansprüche 1 bis 51 aufgetragen wird.